(19) **Europäisches Patentamt / European Patent Office / Office européen des brevets**

(11) **EP 3 368 010 B1**

(12) **EUROPEAN PATENT SPECIFICATION**

(45) Date of publication and mention
of the grant of the patent:
**10.12.2025 Bulletin 2025/50**

(21) Application number: **16860721.6**

(22) Date of filing: **26.10.2016**

(51) International Patent Classification (IPC):
**A61K 9/00** *(2006.01)* **A61K 9/20** *(2006.01)*
**A61K 9/16** *(2006.01)* **A61K 9/70** *(2006.01)*
**A61K 9/46** *(2006.01)*

(52) Cooperative Patent Classification (CPC):
**A61K 9/0007; A61K 9/1635; A61K 9/1641;
A61K 9/2027; A61K 9/2031; A61K 9/2072;
A61K 9/2077; A61K 9/2095; A61K 9/7007**

(86) International application number:
**PCT/US2016/058935**

(87) International publication number:
**WO 2017/075096 (04.05.2017 Gazette 2017/18)**

(54) **SOLID DOSAGE FORM IMMEDIATE DRUG RELEASE AND APPARATUS AND METHOD FOR MANUFACTURE THEREOF**

FESTE DOSIERUNGSFORM FÜR SOFORTIGE WIRKSTOFFFREISETZUNG SOWIE VORRICHTUNG UND VERFAHREN ZUR HERSTELLUNG DAVON

FORME GALÉNIQUE SOLIDE À LIBÉRATION IMMÉDIATE DE MÉDICAMENT ET APPAREIL ET PROCÉDÉ DE FABRICATION ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.10.2015 US 201562246470 P
27.01.2016 US 201614907891
11.07.2016 US 201662360546 P
19.08.2016 US 201662377068 P**

(43) Date of publication of application:
**05.09.2018 Bulletin 2018/36**

(73) Proprietor: **Blaesi, Aron H.
7078 Lenzerheide (CH)**

(72) Inventors:
• **BLAESI, Aron, H.
Dieschen seura 1 (CH)**
• **SAKA, Nannaji
Cambridge, MA 02139 (US)**

(56) References cited:
EP-A2- 0 253 554 WO-A1-94/07470
WO-A1-98/36738 US-A- 4 291 015

US-A1- 2003 021 845 US-A1- 2003 035 833
US-A1- 2004 028 732 US-A1- 2004 166 153
US-A1- 2005 147 670 US-A1- 2011 105 441
US-A1- 2012 219 624 US-A1- 2014 023 708
US-A1- 2014 271 530 US-A1- 2014 356 426
US-A1- 2014 356 426 US-A1- 2015 164 816
US-A1- 2015 246 016

• MOULTON SIMON E ET AL: "3-dimensional (3D) fabricated polymer based drug delivery systems", JOURNAL OF CONTROLLED RELEASE, ELSEVIER, AMSTERDAM, NL, vol. 193, 11 July 2014 (2014-07-11), pages 27 - 34, XP029084255, ISSN: 0168-3659, DOI: 10.1016/ J.JCONREL.2014.07.005
• WOLKERS WF ET AL: "iMedPub Journals Pcl/ PegElectrospunFibersas DrugCarriersfortheControlled DeliveryofDipyridamole", 21 March 2015 (2015-03-21), XP055562957, Retrieved from the Internet <URL:http://pharmacology.imedpub. com/ pclpeg-electrospun-fibers-as-drug-carriers-for- the-controlled-delivery-of-dipyridamole.pdf> [retrieved on 20190228]

**Description**

**Cross Reference to related Co-Pending Applications**

This application claims priority to and the benefit of U.S. Provisional Patent Application No. 62/246,470 filed October 26, 2015 and titled POLYMERIC DOSAGE FORM FOR IMMEDIATE DRUG RELEASE.

This application also claims priority to and the benefit of U.S. Application Serial No. 14/907,891 filed on January 27, 2016 and titled MELT-PROCESSED POLYMERIC CELLULAR DOSAGE FORM.

This application also claims priority to and the benefit of U.S. Provisional Application Serial No. 62/360,546 filed on July 7, 2016 and titled "POLYMERIC CELLULAR DOSAGE FORMS AND APPARATUS AND METHOD FOR CONTINUOUS MANUFACTURE THEREOF".

This application also claims priority to and the benefit of U.S. Provisional Application Serial No. 62/377,068 filed on August 19, 2016 and titled "FIBROUS DOSAGE FORMS FOR IMMEDIATE DRUG RELEASE".

**Field of the Invention**

The present invention relates to solid dosage forms for immediate drug release and an apparatus and a method for manufacture thereof, and more particularly to solid dosage forms that are produced via solidification of a fluid and an apparatus and a method for manufacture thereof.

**Background of the Invention**

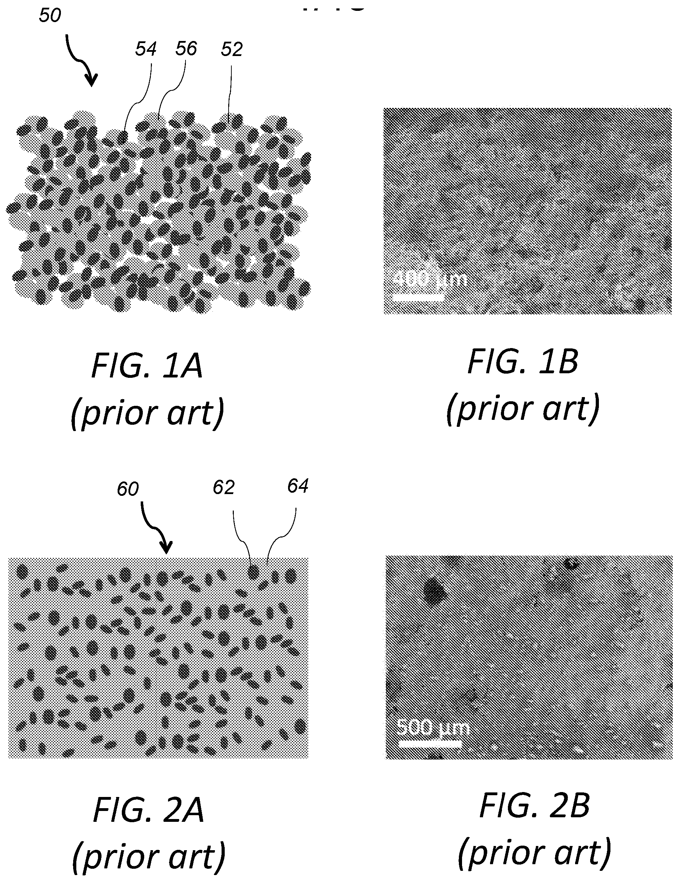

Today, most pharmaceutical formulations are administered in the form of a solid dosage form as orally delivered tablet or capsule. Specifications of such solid dosage forms are highly regulated, and include requirements on drug content, drug dissolution (drug release), and the maximum amount of impurities and degradation products, among others. Currently, solid dosage forms 50 are typically complex mixtures of compressed granules 52 containing pharmaceutically active ingredients (drugs) 54 embedded in pharmaceutically inactive ingredients (excipients) 56, as shown in FIG. 1A. The excipients serve to guarantee that the dosage form meets its specified drug-release profile, to improve processability, and to ensure that the dosage form meets requirements of chemical stability and mechanical strength. Bonding between the granules is primarily achieved by mechanical interlocking of the surfaces. This results in plastic deformation of the granules during compaction. FIG. 1B presents a Scanning Electron Microscopy (SEM) image of the microstructure of a commercially available granular tablet. The individual granules cannot be distinguished or identified within the plastically deformed, compacted matrix.

Nonetheless, a granular dosage form 50 is typically designed to permit some percolation of gastrointestinal fluid to the interior after ingestion, and then the bonds between the granules are ruptured so that the dosage form disintegrates rapidly into its constituent particles. Fast disintegration provides a large specific surface area of the solid drug particles exposed to the gastrointestinal fluid, and consequently promotes rapid drug dissolution. This yields a high concentration of dissolved drug molecules in the upper gastrointestinal tract to ensure that a large fraction of the ingested drug is absorbed by the blood stream and distributed to the disease-specific target sites in the human body.

The manufacturing process is critical for the formulation to achieve the desired properties, and hence for the production of high-quality products. The present standard technologies of manufacturing solid formulations are powder-based and include powder compaction to produce a tablet or powder filling into a pharmaceutical capsule. Extensive preparation of the powder is required for it to possess the desired properties of flowability and compressibility, and to guarantee that performance-related properties are met by the dosage form. Typical process steps to produce the most common pharmaceutical products via particulate/powder processing include blending and granulating, drying, milling and screening, blending, tableting, and coating. Due to the large number of downstream unit operations used to produce solid dosage forms a number of process inefficiencies occur. These process inefficiencies include long process time, high machine and human capital cost, high footprint in the facility, as well as expensive pharmaceutical development, scale-up, and quality control processes, among others. Moreover, because the theories elucidating particulate behavior are still incomplete it is difficult to predict and control manufacturing processes. For example, mixing a drug with the carrier, or excipient, particles is hampered by particle aggregation and segregation, and dispensing and compacting the particulates is complicated by their unpredictable uneven flow. As a consequence, the microstructural properties of the dosage forms are not deterministic. This results in large batch-to-batch variations in product properties, excessive out-of-specification product waste, and the requirement for expensive off-line quality testing. Furthermore, the lead-times for developing and scaling up a formulation are unduly long, limiting flexibility in product development and timely delivery of dosage forms for clinical trials.

Alternative processing technologies for manufacturing solid dosage forms are liquid-based technologies, such as injection molding, hot-melt extrusion, solvent casting, and hot-melt casting, among others. Manufacturing dosage forms by casting or molding may mitigate many of the above mentioned limitations. In the liquid-based process technologies, the material is fluidized either by a solvent or by melting and is handled in liquid form, thus imparting reproducible, predictive microstructure and properties. The microstructure of a cast solid dosage form 60 usually includes active drug particles 62 embedded in a non-porous matrix 64, as schematically shown in FIG. 2A. FIG. 2B presents a SEM image of the microstructure of such a non-porous or minimally-porous cast dosage form. Several studies have shown, however, that cast dosage forms, particularly if they consist of biologically inert and chemically and physically stable polymeric excipients, are appropriate only for long-term or sustained release. They are not suitable for immediate drug release, as cast matrices resist percolation of the dissolution medium, giving a slow rate of drug release. Although the drug release rate of dosage forms based on solid matrices could be increased if the drug is embedded in highly soluble small molecules (e.g., specific types of sugars or polyols), embedding the drug in such materials is typically inferior because they are bioactive and/or difficult to process to a dosage form that satisfies the requirements on chemical stability and mechanical properties, among others.

It would be desirable to have solid dosage forms and an apparatus and a method for continuous manufacture thereof that overcome the above mentioned limitations. There is also a need for solid dosage forms with predictable and flexible drug release properties which can be prepared by a cost-effective, deterministic process with short process time.

## Summary of the Invention

The present invention relates to solid dosage forms and an apparatus and a method for manufacture thereof as defined by the claims. In particular, the present invention relates to solid dosage forms that are produced via solidification of a fluid and an apparatus and a method for manufacture thereof. The solid dosage forms are suitable for immediate drug release applications. The microstructure of the solidified dosage forms includes thin drug-containing elements that are embedded in or attached to an excipient. The drug-containing elements have the form of thin sheets, fibers, or particles.

In general, in one aspect, the invention features a solid pharmaceutical dosage form including a drug-containing solid and a nondrug-containing matrix as defined by the claims. The drug-containing solid includes one or more drug-containing structural elements that are embedded in or attached to the nondrug-containing matrix. The drug-containing structural elements include one of zero-dimensional elements, one-dimensional elements, two-dimensional elements, or combinations thereof. The drug-containing structural elements are a solidified liquid or paste. The pharmaceutical dosage form has a size greater than an average thickness of the one or more drug-containing structural elements.

Implementations of this aspect of the invention may include one or more of the following features. The zero-dimensional elements are particles or beads. The one-dimensional elements are fibers. The two-dimensional elements are sheets. Each of the drug-containing structural elements includes one or more hydrophilic excipients and one or more active ingredients. The one or more hydrophilic excipients include one of polyethylene glycol (PEG), polyvinylpyrrolidone (PVP), PEG-PVP copolymer, poloxamer, hydroxypropyl methylcellulose, lauroyl macrogol-32 glycerides, polymethylmethacrylates, polyvinylacetate phthalate, polyvinyl alcohol (PVA), PEG-PVA copolymer, polybutylmethacrylat-(2-dimethylaminoethyl)methacrylat-methylmathacrylat-copolymer, poly(methacrylic acid, ethyl acrylate) 1:1, polyvinyl caprolactam-polyvinylacetate-polyethylene glycol copolymer, or gelatin. At least one of the one or more hydrophilic excipients is soluble in body fluids and has a solubility greater than 5g/l in body fluids. The dissolved molecules of the soluble hydrophilic excipient have a diffusivity greater than $1x 10^{-8} m^2/s$ in body fluids. At least one of the one or more hydrophilic excipients is swellable in body fluids and the rate of penetration of the body fluids into a solid, swellable excipient is greater than an average thickness of the one or more drug-containing elements divided by 1800 seconds. At least one of the one or more hydrophilic excipients is swellable in body fluids and an effective diffusivity of water in the swellable excipient is greater than $1x10^{-11} m^2/s$. The swellable excipient has a viscosity less than 500 Pa·s upon absorption of the body fluids. The nondrug-containing matrix is one of a solid, a liquid, or a gas. The one or more drug containing structural elements are embedded in the nondrug-containing matrix and the nondrug-containing matrix is partially or entirely removed from the dosage form after contact with a body fluid. The nondrug-containing matrix is a solid, and the solid is bonded to at least one of the one or more drug-containing structural elements or to a segment of the one or more drug-containing structural elements, and the solid has a solubility greater than 50 g/l and dissolved molecules of the solid have a diffusivity greater than $4x10^{-8} m^2/s$ in body fluids. The nondrug-containing matrix is a solid and the solid has a solubility greater than 50 g/l and the dissolved molecules of the solid have a diffusivity greater than $4x10^{-8} m^2/s$ in body fluids. The the solid includes randomly or almost randomly distributed particles and a volume fraction of the particles relative to a representative control volume is greater than about 0.3. The nondrug-containing matrix is a solid and the solid is one of sugars, polyols, polymers, effervescent agents, or combinations thereof. The polyols are one of sucrose, lactose, maltose, glucose, maltodextrin, mannitol, maltitol, isomalt, lactitol, xylitol, erythritol, sorbitol, or combinations thereof. The polymers are one of polyethylene glycol, polyvinyl pyrrolidone, polyvinyl alcohol, or copolymers thereof. The effervescent agents are sodium bicarbonate. The nondrug-containing matrix is a gas and the gas is one of air, nitrogen, $CO_2$, argon, oxygen, or combinations thereof. The nondrug-containing matrix is a liquid and the liquid is one of Polyethylene glycol (PEG) with molecular weight smaller than 1000 Da (e.g. PEG 400, PEG 300, etc.), Poloxamer 124, 2-Pyrrolidone, Glycerol triacetate (Triacetin), D-alpha tocopheryl polyethylene glycol 1000 succinate (TPGS), Polyoxyl Hydroxystearate, Polyoxyl 15 Hydroxystearate, Castor oil, Polyoxyl castor oil (Polyethoxylated castor oil), Polyoxyl 35 castor oil, Polyoxyl hydrogenated castor oil, Glyceryl monooeleate, Glycerin, Propylene glycol, Propylene carbonate, Propionic acid, Peanut oil, Sesame oil, Olive oil, Almond oil, combinations of such liquids with a polymer or any other molecule that dissolves in them. The nondrug-containing matrix serves as core and the drug-containing solid surrounds the core. Part of the drug-containing solid interfaces an outer surface of the dosage form. The nondrug-containing matrix is a solid, and the solid is one of cellulose, cellulose derivatives (microcrystalline cellulose, Hydroxypropyl cellulose, Hydroxyethyl cellulose, Methyl cellulose, Hydroxypropyl methyl ether cellulose), Polylactic acid, Polylactide-co-glycolide, Polyethylene glycol, Polyvinylpyrrolidone, Polyvinylalcohol, polyvinyl caprolactam-polyvinyl acetate-polyethylene glycol graft copolymer, poloxamer, starch, pregelatinized starch, stearic acid, lactose, polymethacrylates, or combinations thereof. The pharmaceutical dosage form has a size greater than 1mm. The one or more drug-containing structural elements have an average thickness between 1 $\mu$m and 2 mm. The one or more drug-containing structural elements are arranged in the nondrug-containing matrix so that an average 'free spacing' between the drug-containing structural elements is greater than 1 $\mu$m. A volume fraction of the drug-containing solid with respect to the total volume of the dosage form is no greater than 0.97. The dosage form has a disintegration time less than 45 minutes. The one or more drug-containing structural elements have different active ingredients. A first drug-containing structural element is bonded to a second drug-containing structural element and at least 20% to 50% of the first drug-containing element's surface interfaces either one or both of (a) the nondrug-containing matrix and (b) a surface of the dosage form. A first segment of a drug-containing structural element is bonded to a second segment of the same drug-containing structural element and at least 20% to 50% of the drug-containing element's surface interfaces either one or both of (a) the nondrug-containing matrix and (b) a surface of the dosage form. A minimum number of walls that must be ruptured to obtain an interconnected, continuous cluster of nondrug-containing matrix from a surface of the dosage form to any point inside the dosage form is between 0 and 10, for one or more drug-containing structural elements having an average thickness greater than 100 $\mu$m. A minimum number of walls that must be ruptured to obtain an interconnected, continuous cluster of nondrug-containing matrix from a surface of the dosage form to any point inside the dosage form is between 0 and 20, for one or more drug-containing structural elements having an average thickness smaller than 100 $\mu$m. The one or more drug-containing structural elements are wrapped up to form a continuous microstructure. The dosage form further includes a coating covering the dosage form's outer surface. The coating has a heterogeneous thickness, and the coating provides mechanical support for the drug-containing structural elements and fast disintegration after immersion in a body fluid for rapid dissolution of the drug. The coating comprises one of polyethylene glycol (PEG), polyvinylpyrrolidone (PVP), PEG-PVP copolymer, polyvinylalcohol (PVA), PEG-PVA copolymer, polylactic acid, poloxamer, hydroxypropyl methylcellulose, lauroyl macrogol-32 glycerides, polyvinylacetate phthalate, poly(methacrylic acid, ethyl acrylate) 1:1 or butylmethacrylat-(2-dimethylaminoethyl)methacrylat-methylmathacrylat-copolymer, or gelatin. The drug-containing solid comprises a layer attached to a surface of the nondrug-containing matrix. The drug-containing solid comprises a layer surrounding a nondrug-containing matrix. The nondrug-containing matrix comprises a comb-structure and the drug-containing solid comprises a layer attached to the comb-structure's outer surface. The dosage form has a tensile strength greater than 0.05 MPa and a yield strength greater than 0.05 MPa. The dosage form further includes another solid that contains a drug, and 80 percent of the other solid's drug content is converted to dissolved molecules in a time greater than 60 minutes after immersion of the dosage form in a body fluid. The dosage form further includes a filler, a stabilizer, a preservative, a taste masker, a sweetener, or any other common excipient. The pharmaceutical dosage form comprises one of a three-dimensional tablet, a capsule, a suppository, a film, or a patch.

In general, in another aspect, the invention features a method for the continuous manufacture of a solid pharmaceutical dosage form as defined by the claims, including the following. First, mixing, fluidizing, and pressurizing a drug and an excipient to form a fluidic or fluid-like, pressurized mixture. Next, attaching a nondrug-containing matrix to the mixture and then solidifying the mixture via cooling or drying to form a pharmaceutical solid dosage form comprising a drug-containing solid and a nondrug-containing matrix. The drug-containing solid includes drug-containing structural elements that are embedded into or attached to the nondrug-containing matrix. The drug-containing structural elements are one of zero-dimensional elements, one-dimensional element, two-dimensional elements, or combinations thereof. The drug-containing structural elements are a solidified liquid or paste. The pharmaceutical dosage form has a size greater than an average thickness of the one or more drug-containing structural elements.

In general, in another aspect, the invention features an apparatus for the continuous manufacture of a solid pharmaceutical dosage form as defined by the claims, including equipment for mixing, fluidizing, and pressurizing a drug and an excipient to form a fluidic or fluid-like, pressurized mixture, equipment for attaching a nondrug-containing matrix to the mixture, equipment for solidifying the mixture via cooling or drying to form a solid pharmaceutical dosage form comprising a drug-containing solid and a nondrug-containing matrix. The drug-containing solid includes drug-containing structural elements that are embedded into or attached to the nondrug-containing matrix. The drug-containing structural elements include one of zero-dimensional elements, one-dimensional element, two-dimensional elements, or combinations thereof. The drug-containing structural elements are a solidified liquid or paste. The pharmaceutical dosage form has a size greater than an average thickness of the one or more drug-containing structural elements.

The details of one or more embodiments of the invention are set forth in the accompanying drawings and description below. Other features, objects, and advantages of the invention will be apparent from the following description of the preferred embodiments, the drawings and from the claims.

**Brief Description of the Drawings**

Referring to the figures, wherein like numerals represent like parts throughout the several views:

FIG. 1A is a schematic diagram of the microstructure of a prior art granular processed dosage form;

FIG. 1B is a scanning electron microscopy (SEM) image of the microstructure of a prior art granular processed dosage form;

FIG. 2A is a schematic diagram of the microstructure of a prior art non-porous melt-processed dosage form;

FIG. 2B is a scanning electron microscopy (SEM) image of the microstructure of a prior art melt-processed dosage form;

FIG. 3A is a schematic diagram of the microstructure of a cellular melt-processed dosage form with closed cells;

FIG. 3B is a schematic diagram of the microstructure of a cellular melt-processed dosage form with open cells;

FIG. 4 is a schematic diagram of the microstructure of one embodiment of a solid dosage form according to this invention;

FIG. 5 depicts schematic diagrams of the microstructure of additional embodiments of solid dosage forms according to this invention;

FIG. 6 shows schematic diagrams of the microstructure of solid dosage forms according to this invention illustrating the 'free spacing' between neighboring drug containing structural elements;

FIG. 7 shows a schematic diagram of the microstructure of solid dosage forms according to this invention illustrating the number of walls that must be ruptured to obtain an interconnected cluster of nondrug-containing matrix that extends from the surface of the dosage form to a point in the interior;

FIG. 8 depicts a schematic diagram of the microstructure of a coated solid dosage form according to this invention;

FIG. 9 depicts schematic diagrams of the microstructure of additional embodiments of solid dosage forms according to this invention;

FIG. 10 presents schematic diagrams of the microstructure of additional embodiments of solid dosage forms according to this invention;

FIG. 11 is a flow diagram of a method for the manufacture of a solidified dosage form from polymeric melts, according to this invention;

FIG. 12 is a diagrammatic view of an apparatus for the continuous manufacture of solidified dosage forms from polymeric melts;

FIG. 13 presents schematic diagrams of bottom-up approaches to manufacture the solid dosage forms of this invention;

FIG. 14 is a schematic diagram of a bottom-up approach to manufacture multi-component solid dosage forms of this invention;

FIG. 15 is a flow diagram of another method for the manufacture of a solidified dosage form from polymeric melts, according to this invention;

FIG. 16 is a diagrammatic view of another apparatus for continuous manufacture of solid dosage forms from polymeric melts;

FIG. 17A-FIG. 17D depict schematic steps of a molding sequence for producing coated solid dosage forms of this invention;

FIG. 18 is a photograph of an apparatus for the continuous manufacture of fibrous dosage forms according to this invention; and

FIG. 19 depicts scanning electron microscopy (SEM) images of dosage forms according to this invention.

## Detailed Description of the Invention

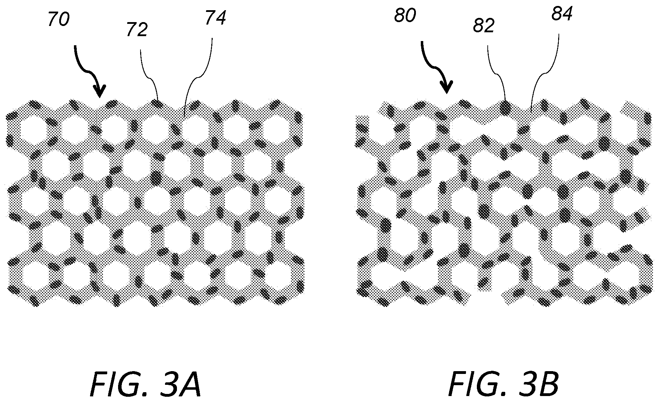

Melt-processed polymeric cellular dosage forms exhibiting improved immediate-release properties, while maintaining high uniformity and satisfactory mechanical properties have been described in co-pending patent application US 14/907,891, entitled MELT-PROCESSED POLYMERIC CELLULAR DOSAGE FORM which is commonly assigned. These polymeric cellular dosage forms have a unique cellular microstructure featuring closed cells 70 and a number of open, interconnected cells 80, as shown in FIG. 3A and FIG. 3B, respectively. The cell walls contain one or more active ingredients 72, 82 as well as an excipient 74, 84 that dissolves or swells in the presence of a physiological/body fluid such as gastrointestinal fluid and/or saliva under physiological conditions.

The present invention provides solid dosage forms that are suitable for immediate drug release applications and are produced via solidification of a fluid. The dosage forms have the outer shape of a three-dimensional tablet or capsule, a suppository, a film, or a patch, among others. However, unlike the microstructure of conventionally solidified (i.e., cast) dosage forms which are minimally-porous solids composed of drug and excipient, the microstructure of the novel dosage forms includes an arrangement/assembly of one or more drug-containing structural elements that are embedded in or attached to a nondrug-containing matrix. The drug-containing structural elements are two-dimensional elements, one-dimensional elements, zero-dimensional elements, or combinations thereof. In mathematical terms, a two-dimensional element is a plane that extends in two-dimensional space; a one-dimensional element is a line that extends in one-dimensional space; and a zero-dimensional element is a point. However, because neither a plane, nor a line, nor a point have a volume or mass, the mathematical definition is relaxed here and adapted for engineering purposes. In the context of this work, a two-dimensional element has two dimensions greater and one dimension smaller than a critical length. For example, the length and width of the element are greater than the critical length, but the thickness is smaller than the critical length. An example of such an element is a "sheet". A one-dimensional element has one dimension greater and two dimensions smaller than the critical length. For example, the length is greater than the critical length, but the width and thickness are smaller than the critical length. An example of such an element is a "fiber". A zero-dimensional element has zero dimensions greater than the critical length. Thus the length, width, and thickness of the element are smaller than the critical length. Examples of such zero-dimensional elements are "particles" or "beads" and include polyhedra, spheroids, ellipsoids, or clusters thereof. The critical length (or thickness) in this work is no greater than 2 mm. Examples of the shapes of the drug-containing structural elements include thin sheets, fibers, particles or beads, or combinations thereof.

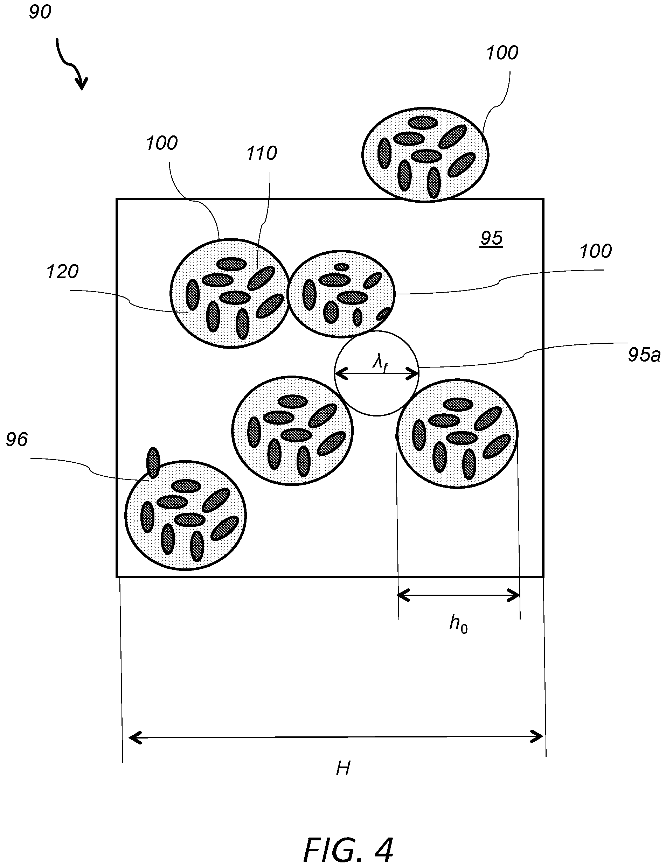

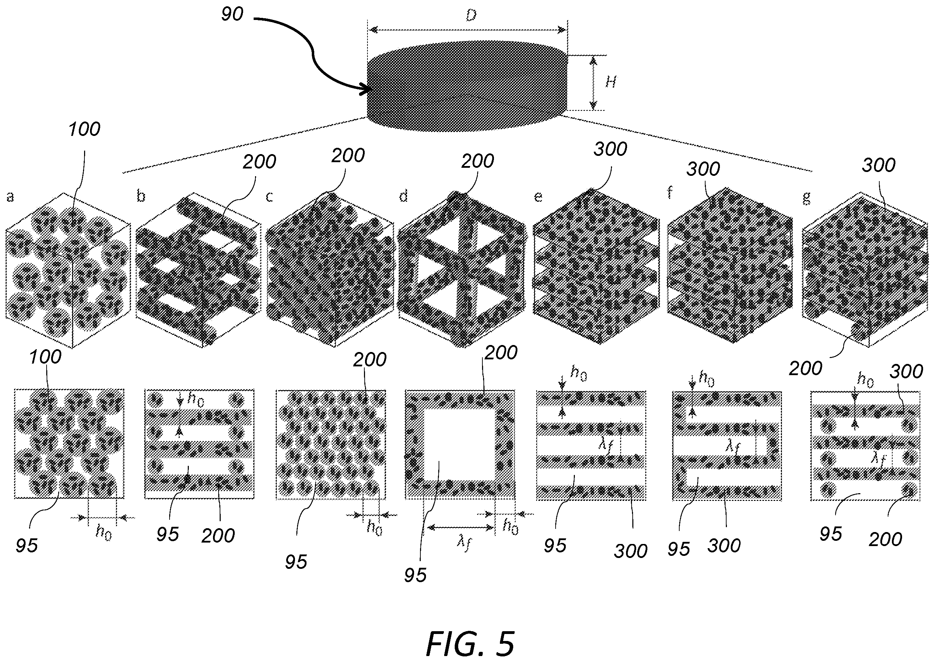

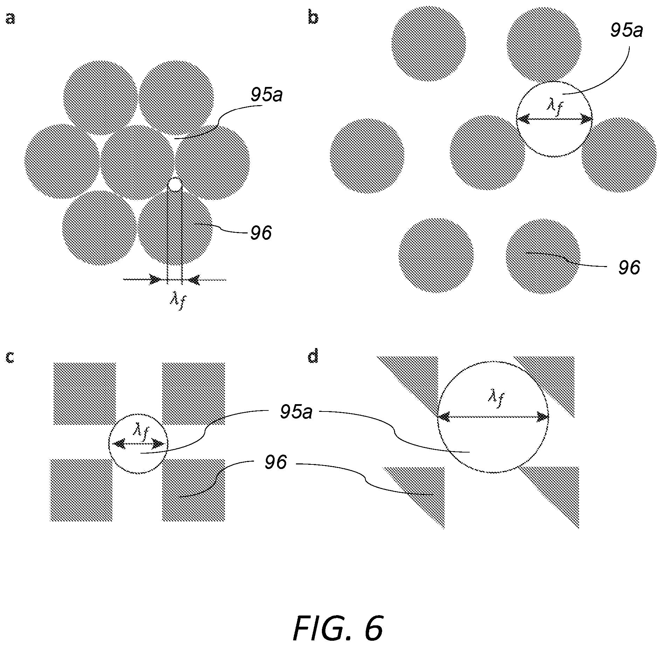

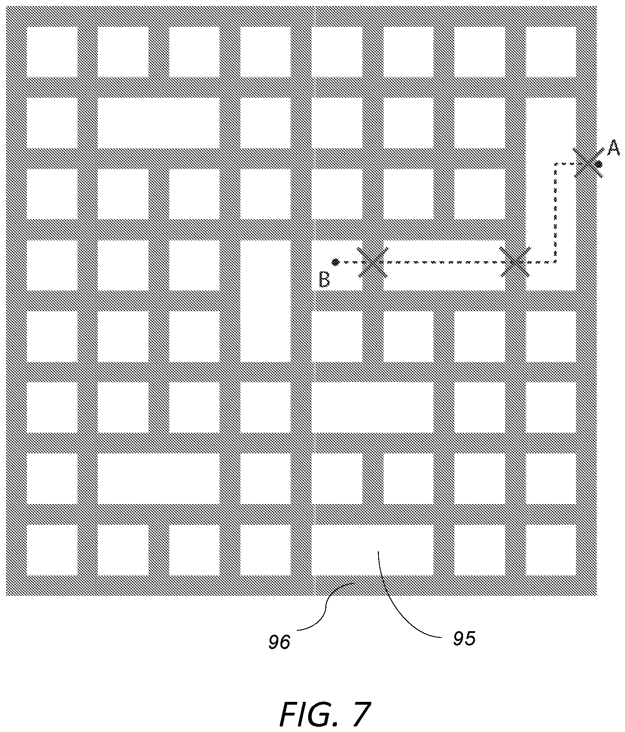

Referring to FIG. 4, and FIG. 5, solidified dosage forms 90, according to this invention, are multi-component, multi-phase materials including a drug-containing solid 96 and a nondrug-containing matrix 95. Drug-containing solid 96 includes drug-containing structural elements 100, 200, 300 that are embedded in or attached to the nondrug-containing matrix 95. The drug-containing structural elements have the geometry of two-dimensional thin sheets 300, one-dimensional fibers 200, zero-dimensional particles or beads 100, or combinations thereof. The cross section of such elements can assume any geometry. Examples of cross section geometries include, but are not limited to, polygons, ellipses, circles, rectangles, and any combinations thereof. Curved surfaces of the cross section may be inward curved (concave) or outward curved (convex). The nondrug-containing matrix 95 is a solid, liquid, or gas, or combinations thereof. The nondrug-containing matrix 95 is either partially or entirely removed from the structure after contact with physiological fluid so that the physiological fluid surrounds the drug-containing structural elements soon after the dosage form is immersed in it. In one example, the solid dosage form 90 has a size greater than 1 mm. The size of the dosage form is considered here the average of the maximum dimension of the dosage form (i.e., the dosage form length or width) and the minimum dimension of the dosage form (i.e., the dosage form thickness). In other examples, the size is greater than one of 1.5 mm, 2 mm, 2.5 mm, 3 mm, 3.5 mm, or 4 mm. A typical dosage form thickness, $H$, is greater than 0.4 mm. In other examples, $H$ is greater than one of 0.7 mm, 1 mm, 1.3 mm, 1.6 mm, 1.9 mm, or 2.2 mm. The one or more drug-containing structural elements 100, 200, 300 have an average thickness $h_0$ no greater than 2 mm. In other examples average thickness $h_0$ is no greater than 1.5 mm, or in the ranges of 1 $\mu$m to 2 mm, 5 $\mu$m to 2 mm, 10 $\mu$m to 2 mm, 15 $\mu$m to 2 mm, 15 $\mu$m to 1.5 mm, 20 $\mu$m - 1.5 mm. The thickness $h$ is considered the smallest dimension of an element (i.e., $h \leq w$ and $h \leq 1$, where $h$, $w$ and $l$ are the thickness, width and length of the element, respectively). The "free spacing" $\lambda_f$ between neighboring elements (or neighboring segments of the same element) on average is greater than 1μm. In other examples, $\lambda_f$ is greater than 2 μm, greater than 5 μm, greater than 7 μm, or in the ranges of 1 μm - 5 mm, 1 μm - 3 mm, 1 μm - 2.5 mm, 1 μm - 2 mm. In the context of this work, the "free spacing" is defined as the maximum diameter of a sphere that fits in the "free" space 95a between neighboring elements (or neighboring segments of the same element) considering the one or more elements as rigid, fixed bodies. The diameter of such spheres can be estimated, for example, from 2-d images of the microstructure. Examples of 2-d representations of such spheres (or circles in 2-d) are given in Fig. 6. As shown in examples a, b, c, and g of FIG. 5, the drug-containing elements 100, 200, 300 may be bonded to another drug-containing element. Furthermore, the drug-containing elements may comprise interpenetrating elements, such as interpenetrating fibers (as in example d of FIG. 5), or interpenetrating sheets (as in the cellular structures presented in FIG. 3A and FIG. 3B). At least 20% to 50% of the drug-containing element surface area, however, interfaces either one or both of (a) the nondrug-containing matrix 95 and (b) the surface of the dosage form (or the dosage form coating in case the dosage form is coated). Part of (or the entire) nondrug-containing matrix 95 may be enclosed by walls comprising the drug-containing solid. If the average element thickness, $h_0$, is greater than 100 μm, an interconnected, continuous cluster of nondrug-containing matrix that extends from the dosage form surface to a given point inside the dosage form is obtained if no more than 0 to 10 (e.g., 0-9, 0-8, 0-7, 0-6, 0-5) walls are ruptured (i.e., walls of drug-containing solid are opened or removed). If $h_0$ is smaller than 100 μm, no more than 0 to 20 (0-18, 0-16, 0-14, 0-12, 0-10) walls must be ruptured to obtain such an interconnected cluster of nondrug-containing matrix. In FIG. 7, a 2-d example is presented that shows 3 walls to be ruptured for obtaining an interconnected cluster of nondrug-containing matrix from point A to point B. Each drug-containing element 100, 200, 300 contains a hydrophilic excipient 120 and drug 110. The drug 110 can be molecularly dissolved in the excipient, or it can be dispersed in the excipient as particles. A solid dosage form 90 can contain multiple different drug-containing elements 100, 200, 300 that have different compositions, different shapes or different sizes. The thicknesses of the drug-containing elements and in some embodiments also the locations where they are placed are fairly repeatable from product to product (i.e., if the same product is made twice). The drug-containing elements can be curved. In this case, it can happen that the neighbor or neighbors of the element at a given location comprise the same element but at a different location (i.e., the neighbor of a specific segment of an element is another segment of the same element). Furthermore, in some embodiments, instead of an assembly of elements, there is only one element that is wrapped up to the desired microstructure (i.e. a continuous fiber or a continuous sheet), as shown in example f in FIG. 5. A typical solid dosage form 90 disintegrates in less than 45 minutes after immersion in a body fluid. In this application, the dosage form disintegration time is considered the time to dissolve 80 percent of the drug content of a dosage form in a USP dissolution test. Drug dissolution must thereby be limited by dosage form disintegration and not by dissolution of the drug particles. The standard deviation in dosage form disintegration time under given conditions is less than 50% of the mean value. Typical standard deviation in drug content is less than 5 wt%.

In one embodiment, the drug-containing elements 100, 200, 300 include at least one excipient 120 that has a solubility greater than 5 g/l in body fluids. In other examples, the solubility of the excipient 120 in body fluids is greater than 10 g/l, or greater than 20 g/l, or greater than 30 g/l, or greater than 50 g/l. For polymers for which solubility data are not available, the 'solubility' in the context of this application is the polymer concentration in water or physiological/body fluid at which the average shear viscosity of the solution is 5 Pa·s in the shear rate range 1-100 1/s at body temperature. The pH value of the physiological/body fluid may thereby be adjusted to the specific physiological condition of interest. The diffusivity of the dissolved excipient molecule in body fluids is greater than $1 \times 10^{-8}$ m$^2$/s. In other examples, the diffusivity of the dissolved excipient molecule in body fluids is greater than $2 \times 10^{-8}$ m$^2$/s, greater than $4 \times 10^{-8}$ m$^2$/s, greater than $6 \times 10^{-8}$ m$^2$/s. Examples of such excipients are polyethylene glycol with molecular weight between 1000 and 600,000 kg/mol. In other examples, excipients are polyethylene glycol with molecular weight between 1500-500,000 kg/mol, or 2000 - 300,000 kg/mol, or 3000 - 100,000 kg/mol. The volume fraction of the soluble excipient in the excipient is greater than 0.02. In other examples, the volume fraction of the soluble excipient in the excipient is greater than 0.04, greater than 0.06, greater than 0.08, or greater than 0.1.

In another embodiment, the drug-containing elements 100, 200, 300 include at least one excipient 120 that is swellable in body fluids. The effective diffusivity of water in this excipient is greater than 1x10-11 m$^2$/s at body temperature. In other examples, the effective diffusivity of water in this excipient is greater than 2x10-11 m$^2$/s, greater than 4x10-11 m$^2$/s, greater than 6x10-11 m$^2$/s, or greater than 8x10-11 m$^2$/s. The viscosity of the swellable excipient after water penetration is less than 500 Pa·s at body temperature. In other examples, the viscosity of the swellable excipient after water penetration is less than 400 Pa·s, less than 300 Pa·s, less than 200 Pa·s, or less than 100 Pa·s at body temperature. In the context of this work, the viscosity is the average shear viscosity of the polymer-water (or polymer-physiological fluid) solution in the shear rate range 1-100 1/s at body temperature. The weight of polymer in the solution is typically 0.1-0.6 (e.g., 0.25-0.5) times the weight of water (or physiological fluid).) Examples of such excipients include enteric materials and are polyethylene glycol, polyvinylpyrrolidone, polyethylene glycolpolyvinyl alcohol copolymers, poly(butyl methacrylate-co-(2-dimethylaminoethyl) methacrylateco-methyl methacrylate) 1:2:1, poly(methacrylic acid, ethyl acrylate) 1:1, polyvinyl acetate phthalate, among others.

As was mentioned above, the nondrug-containing matrix 95 may be a solid, liquid, gas (or vacuum), or combinations thereof. If one or more structural elements (or one or more segments of a structural element) are embedded in the nondrug-containing matrix, the matrix must be removed partially or entirely after contact with dissolution fluid. Examples of a gas matrix 95 that satisfies this requirement include any biocompatible gas, such as air, nitrogen, $CO_2$, argon, oxygen, and nitric oxide, among others. Examples of a solid matrix that is removed or dissolved after contact with physiological fluid 95 include sugars or polyols, such as Sucrose, Lactose, Maltose, Glucose, Maltodextrin, Mannitol, Maltitol, Isomalt, Lactitol, Xylitol, Sorbitol, among others. Other examples of a solid matrix 95 include polymers, such as polyethylene glycol, polyvinyl pyrrolidone, polyvinyl alcohol, among others. Other examples of a solid matrix 95 include effervescent agents, such as sodium bicarbonate. The relevant physical properties of a solid matrix 95 that is bonded to a drug-containing structural element are high solubility and diffusivity in physiological fluids to ensure its rapid removal after contact with physiological fluid. Thus other examples of a solid matrix 95 include solid active pharmaceutical ingredients with high solubility and diffusivity, such as Aliskiren. Typically, a solid material should have a solubility in physiological fluid greater than 50 g/l, 75 g/l, 100 g/l, or 150 g/l, and a diffusivity (as dissolved molecule in physiological fluid) greater than $4\times10^{-8}$ m²/s, $6\times10^{-8}$ m²/s, $8\times10^{-8}$ m²/s, or $1\times10^{-7}$ m²/s to be removed or dissolved rapidly after contact with physiological fluid. Examples of liquids that can surround the drug-containing elements include Polyethylene glycol (PEG) with molecular weight smaller than about 1000 Da (e.g. PEG 400, PEG 300, etc.), Poloxamer 124, 2-Pyrrolidone, Glycerol triacetate (Triacetin), D-alpha tocopheryl polyethylene glycol 1000 succinate (TPGS), Polyoxyl Hydroxystearate, Polyoxyl 15 Hydroxystearate, Castor oil, Polyoxyl castor oil (Polyethoxylated castor oil), Polyoxyl 35 castor oil, Polyoxyl hydrogenated castor oil, Glyceryl monooeleate, Glycerin, Propylene glycol, Propylene carbonate, Propionic acid, Peanut oil, Sesame oil, Olive oil, Almond oil, combinations of such liquids with a polymer or any other molecule that dissolves in them, among others. Furthermore, any of the above listed materials may be combined with a filler material such as microcrystalline cellulose or others, a sweetener, a taste masking agent, a stabilizing agent, a preservative, a coloring agent, other common excipients, and even an active pharmaceutical ingredient.

In other embodiments, nondrug-containing solid matrix 95 serves as a core and the drug-containing solid is attached to the core (for example, it surrounds the core). Examples of solids that can serve as a nondrug-containing core include any solid that is biocompatible, such as cellulose and cellulose derivatives (microcrystalline cellulose, Hydroxypropyl cellulose, Hydroxyethyl cellulose, Methyl cellulose etc.), Polylactic acid, Polylactide-co-glycolide, Polyethylene glycol, Polyvinylpyrrolidone, Polyvinylalcohol, polyvinyl caprolactam-polyvinyl acetate-polyethylene glycol graft copolymer, poloxamer, among others.

As shown in FIG. 4 and in the example a in FIG. 5, the one or more drug-containing elements are particles or beads 100 with thickness, width, and length of the same order of magnitude. Particles 100 are considered as being 0-dimensional elements, and their thickness, width, and length are smaller than the size of the dosage form 90. In the examples b, c, and d in FIG. 5, the one or more drug-containing elements are fibers 200 with thickness and width smaller than their length. Fibers 200 are considered as being one-dimensional elements, and their thickness and width are smaller than the size of the dosage form 90. The fiber length is smaller than, of the order of, or larger than the size of the dosage form 90. In the examples e and f in FIG. 5, the one or more drug-containing elements 300 are sheets with thickness smaller than their width and length. Sheets 300 are considered as being 2-dimensional elements, and their thickness is smaller than the size of the dosage form 90. The width and length of sheets 300 are smaller than, of the order of, or larger than the size of the dosage form. In the example g of FIG. 5, the drug-containing elements include a combination of sheets 300 and fibers 200. If the elements are zero-dimensional or one-dimensional, an open, interconnected space 95 exists between the one or more drug-containing elements. In case some or all of the drug-containing elements are two-dimensional (i.e., sheets), however, closed clusters (or even closed individual cells) of nondrug-containing matrix may exist. Such closed clusters or closed individual cells are entirely surrounded by walls comprising the drug containing solid. An interconnected, continuous cluster of nondrug-containing matrix that extends from the dosage form surface to a given point inside the dosage form is obtained if no more than 0 to 20 walls are ruptured in the dosage forms disclosed herein.

The one or more drug-containing elements are arranged in such a way that the average 'free spacing' between them is greater than 1 μm. The volume fraction of drug-containing elements in the dosage form is no greater than 0.97. In other examples, the volume fraction of drug-containing elements in the dosage form is no greater than 0.95, no greater than 0.93, or no greater than 0.9. Typically, it is in the range 0.1-0.9, depending on how the one or more elements are arranged. For example, a cellular structure with predominantly open-cell topology has a volume fraction of drug containing solid between 0.2 and 0.6. By contrast, a structure that includes closely packed parallel cylindrical elements 200, as shown in example c in FIG. 5 (note that such a structure could also be considered an assembly of sheets if the cylinders are bonded together), has a volume fraction of drug containing solid greater than 0.7. A small volume fraction of drug containing solid is desirable to fill small amounts of drug in a comparable large volume (i.e., if the dosage form is used for delivery of a highly potent drug with a drug dose of just a few milligrams or less). On the contrary, a large volume fraction of drug containing solid is desirable to fill large amounts of drug in a small volume (i.e., if the dosage form is used for delivery of a low potency drug or delivery of multiple drugs with a total drug dose of several 100 mg or more).

The drug-containing elements 100, 200, 300 are composed of one or more pharmaceutically active ingredients (APIs) and one or more excipients (just noted as "excipient" here). The excipient is hydrophilic, so that for the pure excipient material the following equation is valid (if the pore size is on the micro-scale):

$$t_{perc} = \frac{2l_{perc}^2 \mu_f}{\gamma r \cos \theta} < t_{diss} \qquad\qquad (1)$$

where $\mu_f$ is the viscosity of the dissolution fluid, $\gamma$ the surface tension of the dissolution medium, $\theta$ the contact angle, $l_{perc}$ is of the order of the size of the dosage form, r is the radius of the pores, and $t_{diss}$ is the desired dissolution time. Typically, $t_{diss}$ must be no greater than 30 minutes in an immediate-release dosage form (i.e., 80% of the drug content must be dissolved in 30 minutes in a USP dissolution test). Furthermore, the excipient is either soluble in the dissolution fluid and/or it is swellable by the dissolution fluid. In case the excipient is soluble but not swellable by the dissolution fluid, the following equation must hold (if there is no convection):

$$t_{er} = \frac{h_0}{2}\left(\frac{1}{t}\int_0^t j_p \, dt\right)^{-1} = \frac{\pi}{8}\left(\frac{\rho_s}{c_0}\right)^2 \left(\frac{h_0^2}{D_p}\right) < t_{diss} \qquad\qquad (2)$$

where $c_0$ is the solid/liquid interface concentration (i.e., the solubility of the excipient in the dissolution fluid), $D_p$ is the diffusivity of excipient molecules in the dissolution fluid, $\rho_s$ is the density of the solid excipient, and $h_0$ is the minimum of the length, width, or thickness of the one or more elements. Thus given the desired dissolution time, $t_{diss}$, and an excipient with certain properties, the maximum acceptable value $h_0$ can be determined by rearranging Eq. (2). For most excipients and dissolution times of practical importance considered here, $h_0$ must be no greater than 1.5 mm. For the case that the excipient is swellable, the minimum of the length, width, or thickness of the one or more elements is approximately (for systems that follow Fick's law of diffusion) $h_0 = l_{pen} < \text{sqrt}(t_{pen} *D)$, where $D$ is the effective diffusivity of the dissolution fluid in the solid material, and $t_{pen}$ is the desired penetration time (of the order of the desired dissolution time).

The one or more drug-containing elements can be either bonded to each other or they can be arranged in a way so that they do not bond. A low bond strength between the drug-containing elements is desirable for rapid dosage form disintegration after dissolution fluid percolated into the structure. Drug-containing elements that are not bonded or weakly bonded are exfoliated and dispersed in the dissolution medium soon after the dissolution fluid entered the structure. This provides a large surface area-to-volume ratio of the solid constituents for rapid release of the drug molecules into the dissolution medium. A large bond strength, however, is desirable for the dosage form to achieve good mechanical strength. In case the drug-containing elements are bonded (i.e., the elements remain interconnected) after dissolution fluid entered the structure, the bonds must be weakened by the dissolution fluid. This occurs by either dissolution or by swelling (i.e., transition from solid-like to liquid-like) of the bonds. Exfoliation occurs if the bonds are weakened so much that they are unable to resist the external forces applied by the dissolution fluid. The bonds are weakened sufficiently if the shear viscosity of the fully swollen (i.e., saturated) excipient material is smaller than about 200 Pa·s - 500 Pa·s. In other examples, the bonds are weakened sufficiently if the shear viscosity of the fully swollen (i.e., saturated) excipient material is smaller than about 100 Pa·s, or 50 Pa·s, or 10 Pa·s. A fully swollen excipient (i.e., a saturated excipient or an excipient after absorbing dissolution fluid) is considered a solution of swellable excipient and dissolution fluid (i.e., water or physiological fluid at a pH value specific to the condition of interest) at a temperature of 37°C. The weight of swellable excipient in the solution is equal to the weight of swellable excipient in the dosage form. The volume of water in the solution is equal to the volume of nondrug-containing matrix (specifically the volume of nondrug containing matrix that is removed from the dosage form upon contact with dissolution fluid) plus the volume of swellable excipient in the dosage form). Disintegration of the structure occurs, however, not only by exfoliation. To a large part it is also by dissolution of the excipient in the interior of the structure. Excipients that can be used to produce the dosage forms disclosed herein include, but are not limited to polyethylene glycol (PEG), polyvinylpyrrolidone (PVP), PEG-PVP copolymer, poloxamer, hydroxypropyl methylcellulose, lauroyl macrogol-32 glycerides, polymethylmethacrylates, polybutylmethacrylat-(2-dimethylaminoethyl)methacrylat-methylmathacrylat-copolymer, or gelatin, among others. In case the drug containing structural elements are well bonded to each other (or to a solid nondrug-containing matrix), the greater of the tensile strength or yield strength of the assembled dosage form material is no less than 0.05 MPa. In other examples, the greater of the tensile strength or yield strength of the assembled dosage form material is no less than 0.01 MPa, or 0.015 MPa, or 0.02 MPa, or 0.025 MPa, or 0.04 MPa, or 0.06 MPa, or 0.1 MPa, or 0.25 MPa, or 0.5 MPa. Bonding between the drug containing structural elements (or between the structural elements and the nondrug containing matrix) can, for example, be by interdiffusion of molecules, mechanical interlocking, or by other forces due to the surface energy of the materials. In some embodiments, good bonding is achieved without deforming the drug containing structural elements plastically in the solid state. In this case, the drug containing structural elements can be readily distinguished from the nondrug containing matrix, for example by imaging (e.g., SEM), computerized tomography, x-ray, or spectroscopic techniques.

The mechanical properties of some structures disclosed herein (particularly the structures with weakly bonded elements) can be improved, for example, by having a 2-dimensional element that spans the surface of the dosage form.

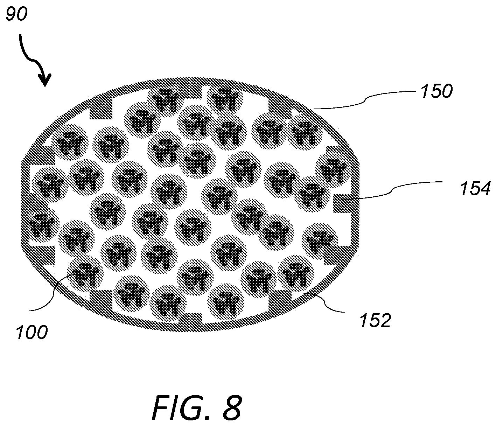

This 2-dimensional element can be a coating material 150, as shown in FIG. 8, or it can be a pharmaceutically active solid composed of drug and excipient. The coating 150 must not only provide mechanical support to the structure, it must also allow its fast disintegration or dissolution. To combine both requirements, the thickness of the coating can be heterogeneous, as shown in FIG. 8. Another option is to fill the zero-dimensional elements, one-dimensional elements, or two-dimensional elements into a capsule (or coating) with uniform thickness. Yet another option is to have a thin layer of the drug and excipient material around the core. In the example of FIG. 8, a coating 150 includes "thick" rings 152 that provide mechanical support and "thin" sheets 154 that disintegrate rapidly after the dosage form is immersed in the dissolution medium. Also the coating materials include, but are not limited to polyethylene glycol (PEG), polyvinylpyrrolidone (PVP), PEG-PVP copolymer, poloxamer, hydroxypropyl methylcellulose, lauroyl macrogol-32 glycerides, or butylmethacrylat-(2-dimethylaminoethyl)methacrylat-methylmathacrylat-copolymer, or gelatin.

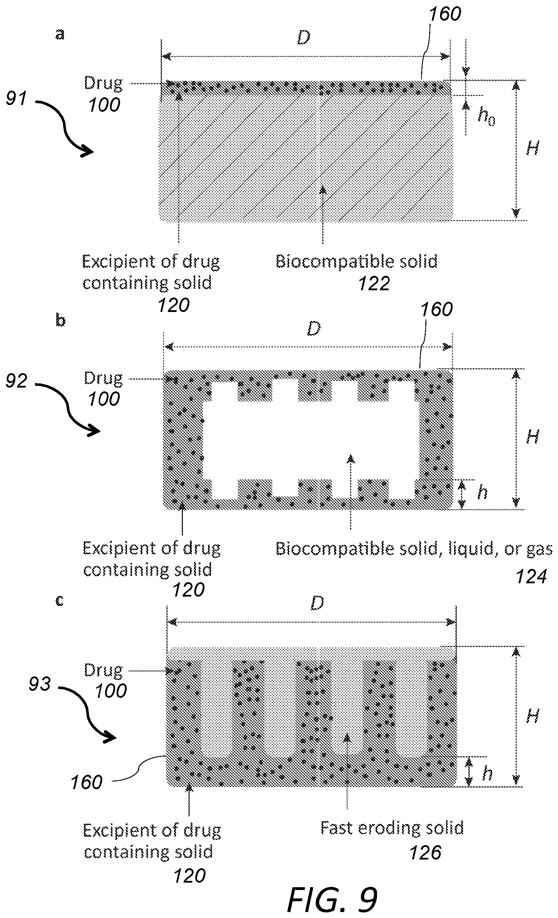

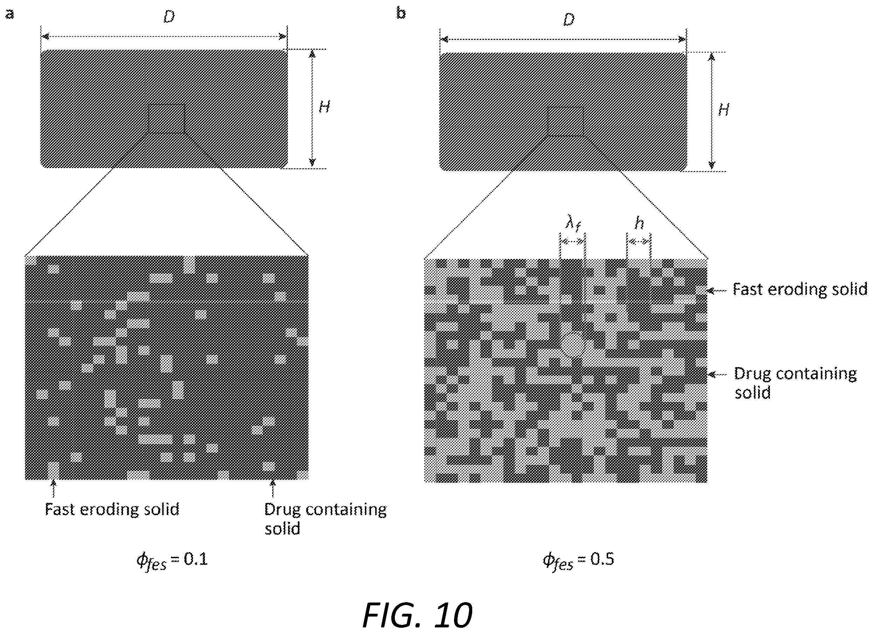

Alternative dosage form structures are shown in FIG. 9 and FIG. 10. In the example (a) of FIG. 9, the dosage form 91 includes a thin layer 160 of drug-containing solid attached to a biocompatible solid (i.e., the nondrug-containing matrix). In some examples, the dosage form 91 contains multiple APIs. The thin layer 160 of drug-containing material includes the drug-containing particles 110 (and/or drug molecules) embedded in the excipient 120. In the example (b) of FIG. 9, layer 160 surrounds a hollow shell structure 124. In the example (c) of FIG. 9, dosage form 93 includes a drug-containing layer 160 that interfaces with a fast eroding excipient 126 having a comb-structure. The comb-structure of the fast eroding solid 126 provides increased interfacial surface area between the drug-containing layer 160 and the fast eroding excipient 126. The fast eroding solid 126 dissolves rapidly after the dosage form is immersed in a dissolution medium (examples of the fast eroding solid include a sugar, a polyol, or an effervescent agent; see above for examples of materials of nondrug-containing matrix surrounding a drug-containing element). In the example (a) of FIG. 10, fast eroding solid particles are randomly or almost randomly distributed in the dosage form at a volume fraction of 0.1. The particles are mostly isolated and do not form interconnected clusters. If the volume fraction of fast eroding solid particles is increased to 0.5, however, as shown in the example (b) of FIG. 10, the particles form interconnected clusters. Structural elements of drug-containing solid can be identified comprising both a thickness and a 'free-spacing' between the elements (or different sections of the same element). Typically, the volume fraction of fast eroding solid with respect to a representative control volume comprising drug-containing solid and nondrug-containing matrix must be greater than about 0.3 to obtain interconnected clusters that are sufficiently large. Such random structures are, however, inferior to the ordered structures presented above. First, large amounts of the fast eroding solid are required. And second, the structural features and dosage form properties are less repeatable and predictable than the ones of the ordered structures.

All the designs introduced here also allow the production of dosage forms with multiple APIs. Furthermore, in other embodiments, some space of the dosage form is occupied by a drug containing solid that releases drug over a prolonged period of time.

In summary, the dosage forms 90, 91, 93, 94 with the one or more elements are structured in such a manner that the mass transfer step, which determines the time of drug release, is reduced from as large as the size of the dosage form (the case of cast dosage form) to as small as the thickness of an element. This results in achieving drug release rates (and drug dissolution times) of solidified dosage forms beyond the limitations of conventionally solidified cast dosage forms.

*Methods and Apparatuses for the manufacture of the solid dosage forms*

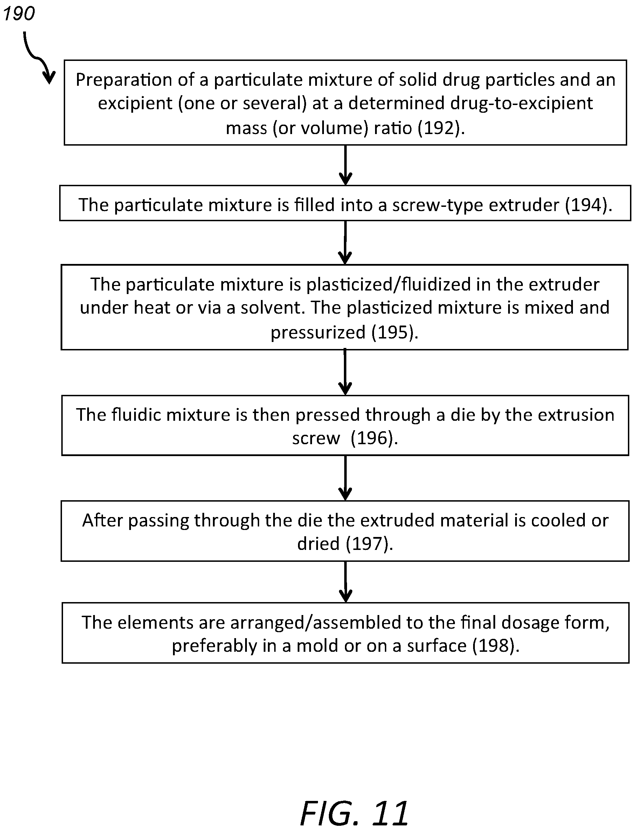

The solid dosage forms presented here are manufactured by solidification of a fluid. The fluid may be either a melt of an excipient and a drug or a fluid (e.g., a paste) including a solvent, an excipient, and a drug. Referring to FIG. 11, in one embodiment, the present invention provides a method 190 of continuous manufacture of solid dosage forms that includes the following steps. First, an excipient 120 and a drug 110 are dosed into an extruder (192), (194). In the extruder, the excipient material is plasticized under heat or via a solvent, and is simultaneously mixed with the drug and pressurized (195). Examples of solvents that are used include water, ethanol, isopropanol, and ethyl acetate, among others. In some cases, the drug and excipient is pre-mixed before dispensing into the extruder. The fluidic mixture is then pressed through a die by the extrusion screw (196). In some embodiments, the die has the same (or a similar) shape as the cross section of the drug-containing element. After passing through the die the extruded material is cooled or dried (197). In one example, the extruded material is cooled with a gas such as cold air or dried by evaporating the solvent. The drug-containing elements are simultaneously arranged/assembled to the final dosage form, preferably in a mold (198) or on a surface. As the elements are arranged/assembled, they can either still be in a fluidic or semi-solid state, or they can be fully solidified in the solid state. In one example, the elements are fluidic or in the semi-solid state (i.e., not fully solidified) when the temperature of the element is still of the order of or above the melting temperature of the excipient. In another example, the elements are fluidic or in the semi-solid state when the amount of solvent in the element is still substantial. If the one or more elements are fluidic or in the semi-solid state prior to assembly, bonding occurs at the contact of two elements (or the contact of two segments of the same element). In this case, bonding may, for example, be due to interdiffusion of fluidic polymer molecules or other means of minimization of surface energy. For such bonding mechanisms, the bond strength is controlled by the temperature-time profile (and the solvent concentration-time profile) of the contact between two elements. If either the full element or the surface of the element is solidified prior to assembly, no bonding or only weak bonding occurs via the mechanisms of interdiffusion and minimization of surface energy. Bonding between two or more elements may, however, be achieved by mechanical interlocking of the elements or by application of appropriate glues in this case.

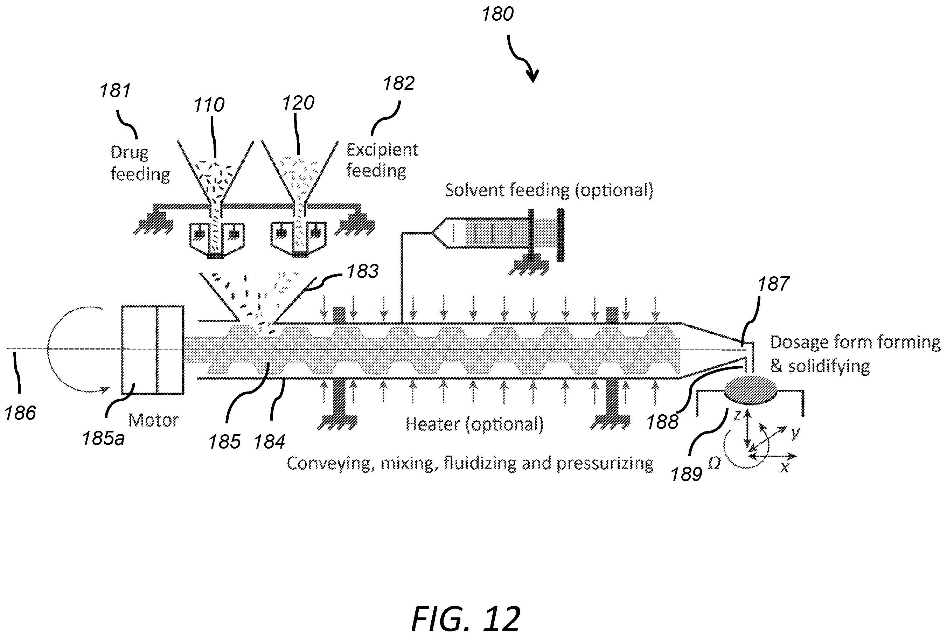

Referring to FIG. 12, an apparatus 180 for the continuous manufacture of the solid dosage forms includes a drug dosing feeder 181, an excipient dosing feeder 182, a solvent dosing system if the dosage forms are fluidized by a solvent, a heater if the dosage forms are fluidized by melting, a screw-type extruder 184 extending along direction 186, a die 187, a nozzle 188 and a mold 189 on a movable linear or rotary stage. Examples of solvents that are used for fluidizing the dosage form ingredients include water, ethanol, isopropanol, and ethyl acetate, among others. As was mentioned above, an excipient 120 and a drug 110 are dosed into an extruder 184 via the drug dosing feeder 181 and the excipient dosing feeder 182, respectively. In the extruder 184, the excipient material is plasticized under heat or via a solvent, and is simultaneously mixed with the drug and pressurized. The fluidic mixture is then pressed through the die 187 by the extrusion screw 185 that is driven by a motor 185a.

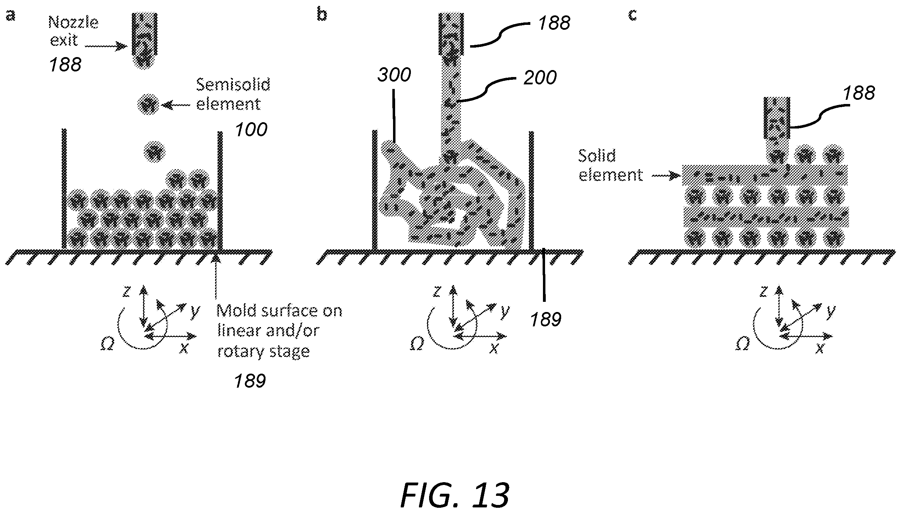

A few illustrative examples of nozzle 188 and mold 189 to build the dosage forms disclosed herein are shown in FIG. 13. In the example (a) of FIG. 13, drug-containing elements 100 having the form of zero-dimensional "particles" are generated and assembled. The "particles" are generated after the fluid stream exits the nozzle and the particles are then stacked to form the dosage forms. "Particle" generation occurs by such 'active' methods as breaking up the fluid stream by exciting the fluid stream with pressure waves or any other form of atomization, by cutting the fluid stream with a knife, blade, or laser, or by stopping the fluid stream with a fluid valve, among others. In one example, pressure waves are generated by a piezo electric actuator. In another example, a fluid valve is 'open' for a short time to deposit a zero-dimensional element and then 'closed' to position the nozzle to another location where another element is deposited. In yet another example, the nozzle velocity with respect to the deposited material (or mold surface) is slow (or even zero) during deposition of a zero-dimensional element. Then as the nozzle (or mold surface) is repositioned to another location to deposit another element, the velocity and travel distance to do so are so large that the fluid stream breaks up. We should further note that 'passive' methods may also be employed to break up the fluid stream. In one example, the properties and velocity of the fluid stream, and the diameter of the nozzle are determined so that the fluid stream that exits the nozzle breaks up due to the Rayleigh instability. In the example (b) of FIG. 13, fibers 200 or sheets 300 are generated and assembled. In one example, assembly of the fibers 200 and sheets 300 occurs by randomly (or almost randomly) knotting or crumbling up the fibers or sheets. The fibers 200 and sheets 300 may also be cut by breaking up the fluid stream with pressure waves, by cutting the fluid stream with a knife, or laser, or by stopping the fluid stream with a fluid valve (for example a needle valve), among others. In other examples, generation and assembly of particles 100, fibers 200 or sheets 300 may be done using multiple nozzles 188. Furthermore, in yet other examples, either the surface of the one or more elements or the entire element is solidified prior to assembly. Thus the elements do not bond to each other or only weakly bond together. In this case, the elements may, for example, be filled in a coating shell (or capsule) which holds the elements in place. The need for compression of the elements is avoided that way. Also in such examples the elements 200, 300 may be cut by breaking up the fluid stream with pressure waves, by cutting the fluid stream with a knife, a laser, or the edge of a mold or a coating shell, or by stopping the fluid stream with a fluid valve, among others.

In any example included in this application, filling, stacking, knotting, and crumbling of the elements 100, 200, 300 can occur with one or multiple nozzles delivering the drug and excipient materials to form the dosage form. In some examples, the nozzles move horizontally and/or vertically, and thus achieve precise computer-control of the location where material is deposited. In other examples, mold 189 is moved and thus computer-control of the deposition location is achieved. In the example (c) of FIG. 13, the mold is moved with a (rotary) x-y-z stage and the nozzle is fixed. The vertical position of the mold is controlled so that the nozzle exit is in close distance to the top surface of the deposited material. This guarantees precise control of where the material is deposited (or supplied). Furthermore, the nozzle 188 may be equipped with equipment to break up the fluid stream, such as piezo electric printheads, fluid valves (for example needle valves), or cutters, among others. Moreover, in any example included in this application, the equipment to break up (or stop) the fluid stream may be controlled by sensors that provide a measure of the amount of material that has been deposited (or injected). Such sensors include pressure sensors or flow meters, among others. The fluid stream may be broken up (or stopped) if the dosage form's specified drug content has been deposited.

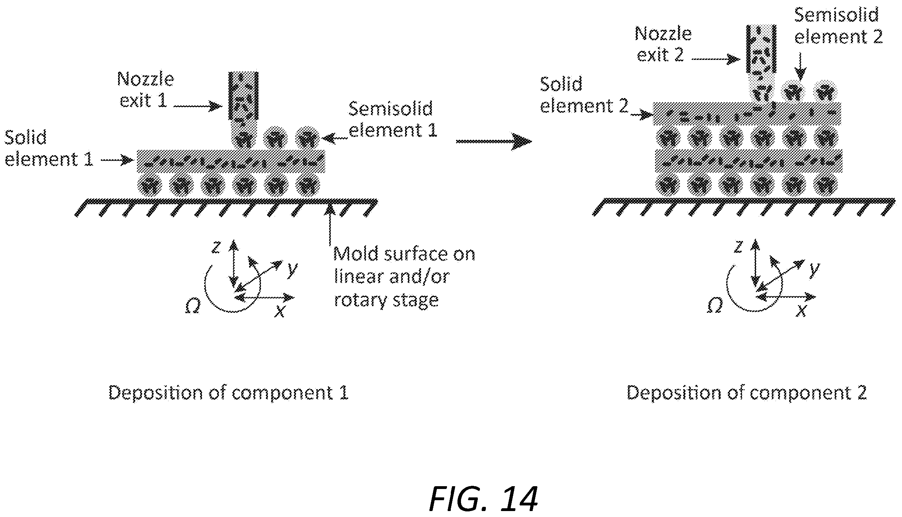

As shown in the example of FIG. 14, an apparatus or method as presented in FIG. 12 and FIG. 13 may be readily adapted to enable manufacture of multi-component products. In one example, component 1 is first deposited by nozzle 1. Then component 2 is deposited by nozzle 2. In another example, a nozzle supplying component 1 may be surrounded by another example supplying component 2. Elements with heterogeneous composition of the cross section may be produced that way.

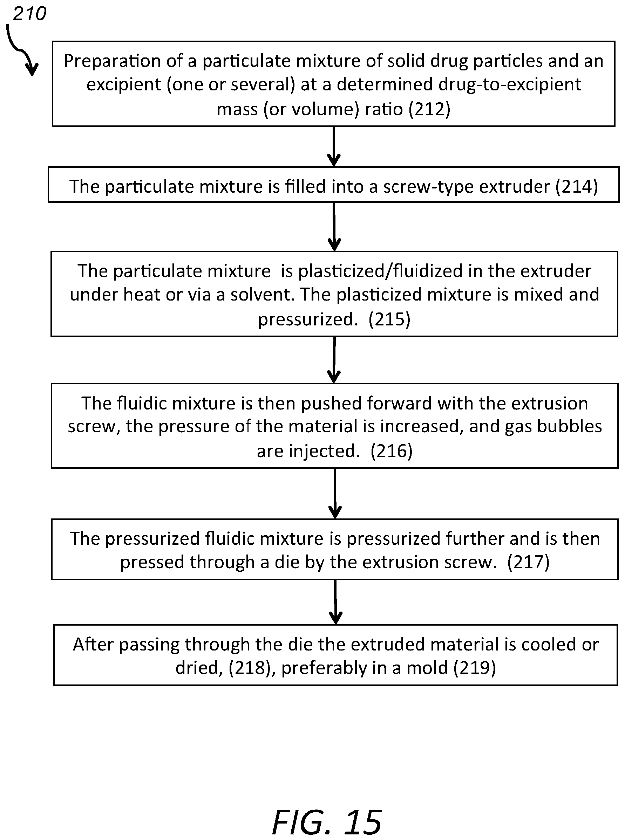

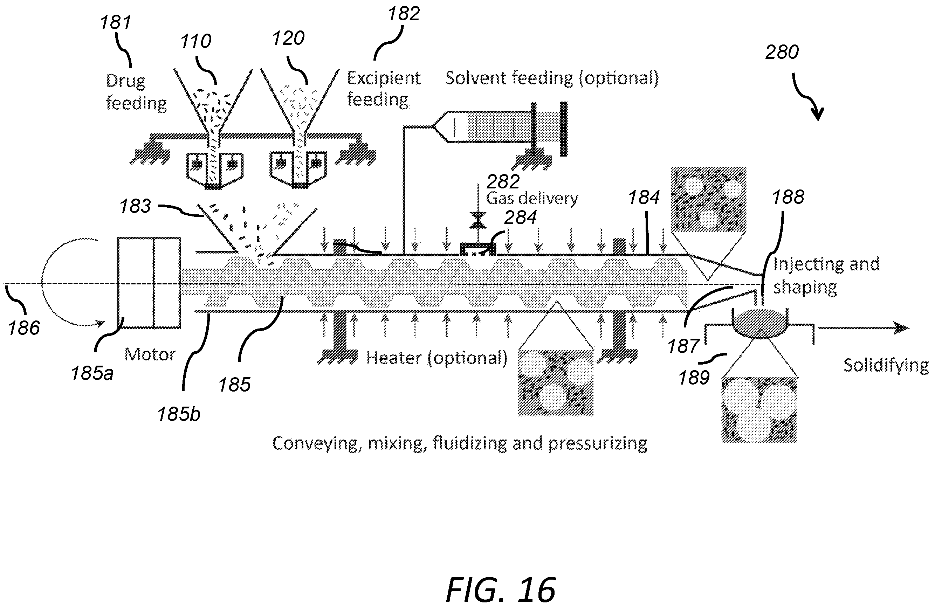

Referring to FIG. 15 and FIG. 16, in another embodiment, a method 210 of manufacture of the solid dosage forms includes the following steps. First, an excipient 120 and a drug 110 are dosed into an extruder 184 (212), (214). In the extruder, the excipient material is plasticized under heat or via a solvent, and then is mixed with the drug and pressurized (215). In some cases, the drug and excipient is pre-mixed before dispensing into the extruder. The fluidic material is then pushed forward along direction 186 by driving the extrusion screw 185 with the motor 185a. As the material is transported forward by the extrusion screw 185, the pressure of the material is increased. At a given location from the inlet of the material into the extruder (and so at a pressure larger than the pressure at the extruder inlet), gas bubbles are supplied to the plasticized material via a gas injection system 282. The gas bubbles are supplied with one or more nozzles 284 of a diameter, $D$, exposed to a gas on one end and to the plasticized material in the extruder on the other end. The gas may be nitrogen, CO2, argon, or air, among others. The diameter of the nozzle 284 is critical, as it affects the size of the bubbles produced. The size of the gas bubbles is tailored by tailoring the nozzle geometry and other parameters, such as the gas pressure, the pressure of fluidized drug and excipient, etc. In one example, a gas nozzle diameter $D$ of 1 $\mu$m - 1 mm is used. The gas delivery pressure is slightly greater than (or about equal to) the pressure of the material at the location of gas delivery. If the pressure of the fluidized drug and excipient material at gas delivery is increased, the amount of gas contained in a given bubble size is increased. A large amount of gas in a bubble gives more opportunities to control the growth of the bubbles later on. A low gas delivery pressure is, however, desirable for ease of processing. In one example, a gas delivery pressure of about 50 psi - 1000 psi is used. Following gas delivery, the rotating extrusion screw further mixes drug with excipient. It also provides mixing of gas bubbles with the plasticized drug and excipient material. Moreover, the extrusion screw pressurizes the material further. This causes the gas bubbles to shrink and some of the gas to dissolve in the melt. The pressurized, mixed material is then dispensed through a nozzle into a mold (217). At the exit of the nozzle, the pressure of the material is reduced to a value lower than the maximum pressure the material has been exposed to at the end of the extrusion screw. This causes the gas bubbles in the mixture to expand. By controlling the delivery pressure of the gas, the flow rate of gas delivery, the gas delivery nozzle geometry, the number of gas delivery nozzles, the location where the gas bubbles are delivered, the maximum pressure of the material in the extruder, the pressure the material is exposed to in the mold, and the cooling rate (or drying rate) of the material in the mold, the microstructure of the resulting dosage form is controlled. The maximum pressure the material is exposed to is set by controlling the screw geometry, particularly the length of the screw 185 and the channel height between screw and the extruder barrel 185b. The microstructural parameters of the resulting dosage form that are affected by these process parameters include the number of cells, the cell size (equivalent to the diameter of voids), the volume fraction of voids (equivalent to the cell volume fraction), the fraction of open cells (equivalent to the fraction of interconnected cells), and the cell wall thickness. Finally, the material is shaped and solidified in the mold by either cooling it below its melting temperature (218) or by drying.

It may be noted that for controlling the amount of material injected into an open or closed mold, injection may be controlled and monitored by sensors that provide a measure of the amount of material that has been injected. Such sensors include pressure sensors or flow meters, among others. The fluid stream may be broken up (or stopped) if the dosage form's specified drug content has been injected in the mold.

FIG. 16 depicts a schematic diagram of an apparatus 280 used for the manufacture of the dosage forms according to the process 210 of FIG. 15. In this embodiment, the apparatus 280 includes a drug dosing feeder 181, an excipient dosing feeder 182, a solvent dosing system if the material must be fluidized by a solvent, a heater if the material must be fluidized by melting, a screw-type extruder 184 extending along direction 186 in a barrel-shaped reactor 185b, a gas injection system 282, gas injectors 284, a die 187, a nozzle 188 and a rotating (or movable) mold 189. As was mentioned above, an excipient 120 and a drug 110 are dosed into an extruder 184 via the drug dosing feeder 181 and the excipient dosing feeder 182, respectively. In the extruder 184, the excipient material is plasticized under heat or via a solvent, and then is mixed with the drug and pressurized. At a given location from the inlet of the material into the extruder, gas bubbles are supplied to the plasticized material via the gas injection system 282. The pressurized fluidic mixture is then pressed through the die 187 by the extrusion screw 185 that is driven by a motor 185a. The mold is filled with the effluent extrudate until the amount of drug in the mold (i.e., the dosage form) assumes the target value. The mold can be open or closed during filling. Finally, the material is solidified by cooling or drying.

The advantage of the disclosed process 210 is that it does not rely on the nucleation of gas bubbles to produce a desired microstructure. Nucleation often requires long time (i.e., 5 seconds or more), high pressure differences (i.e., 800 psi or more), and high temperatures (i.e., 100 °C or more) to provide a sufficient number of gas bubbles for a cellular dosage form. In the present process, gas bubble expansion and microstructure formation in the dosage form occurs in a few seconds or less. It is well controllable and can be done at benign pressures (i.e., a gas delivery pressure of the order of 50 psi to 500 psi (or 50 psi - 1000 psi)) and benign temperatures (i.e., just above the melting temperature of the excipient (or the solvent in a solvent-based process)). In one example, PEG 20k or PEG 35k are used as an excipient, and the process temperature is 70 °C.

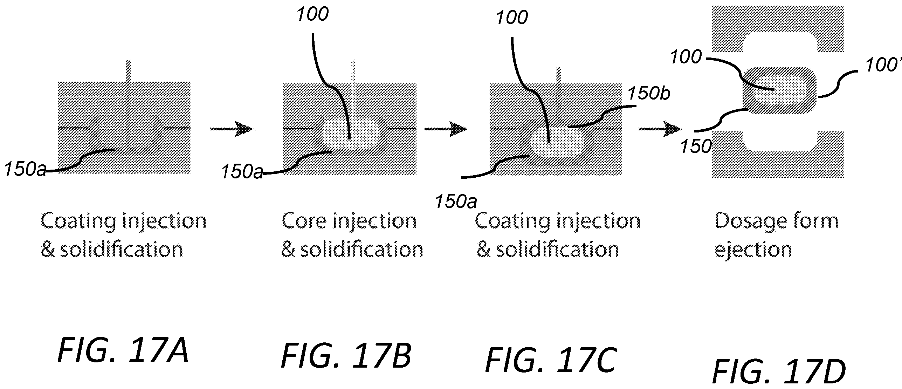

The ease to manufacture multi-component products by liquid-based processing further allows the production of multi-layer, multi-component dosage forms. One aspect of a multi-component dosage form is a coated dosage form. Both the manufacture of the coating and the manufacture of the pharmaceutical core can be integrated into the process, as long as the coating material has the required functional properties (i.e., dissolution time, moisture barrier properties, appearance, color, taste, etc.). In one example, a technology for producing multi-component molded products is overmolding. Dosage form molding with integrated coating by overmolding is done by using a variety of mold technologies, such as core pull-back technologies, rotary molding technologies, rotary cube molding technologies, or movable x-y-z stages. An example of the sequence of coating dosage forms by overmolding using the apparatus of FIG. 16 is shown in FIG. 17A-17D. First, the first half of the coating material 150a is injected and solidified, as shown in FIG. 17A. The first coating half 150a is produced using the first top mold geometry on the side with a single coating injection unit. Next, the core material 100 is overmolded on top of the coating 150a, as shown in FIG. 17B. The mold is rotated (or moved) to the side where the API-containing material is injected. Next, the second half of the coating material 150b is overmolded to produce the coated dosage form 100', as shown in FIG. 17C. The coated dosage form 100' includes the dosage form 100 and the coating 150. Finally, the coated dosage form 100' is elected from the mold, as shown in FIG. 17D.

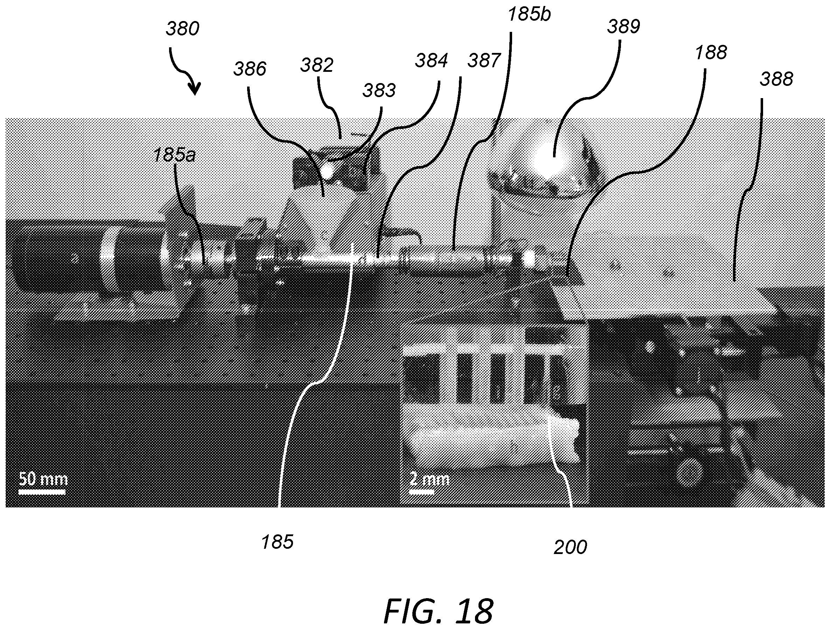

Referring to FIG. 18, an apparatus 380 for the continuous manufacture of fibrous dosage forms 200 includes a granule feeding unit 382, an extrusion screw 185 and barrel 185b, a nozzle 188 where the fibrous melt (or paste) exits, and an x-y-z stage 388 for building of the fibrous structure. The granule-feeding unit 382 includes a syringe 383 with uniform barrel diameter and a syringe pump 384 to deliver the granules into a hopper 386. The volumetric flow rate is controlled by the velocity control of the syringe's piston, as described in U.S. provisional application number 62/360,546, filed on 7/11/2016 and entitled "Polymeric cellular dosage forms and apparatus and method for continuous manufacture thereof".

The hopper 382 directs the granules into the extrusion screw 185 and barrel 185b. Additionally, a solvent feeding system may feed solvent into the extruder if the material must be fluidized by a solvent. In one example, the extrusion screw 185 is 244 mm long, has an outer diameter of 10 mm, a helix angle of 17.65°, a channel height of 1 mm, and a channel width of 8.5 mm. The barrel 185b is surrounded by a resistance heater coil 387 to set the temperature of the barrel. The material effluent from the extruder is delivered into a nozzle 188 with 0.5 mm diameter. In one example, the nozzle 188 is made of Plexiglas and is heated by an infrared lamp 389. The material exiting the nozzle flows downward into the atmosphere and is then assembled and cooled (or dried) to form a fibrous dosage form using an x-y-z stage 388.

It may be noted that the dosage forms could also be manufactured by a 3-d printing process using commercially available equipment. The methods and equipment disclosed herein, however, present process technologies that integrate multiple process steps and further enable substantially greater process rates, smaller footprint, and less complexity than any technique that is presently available.

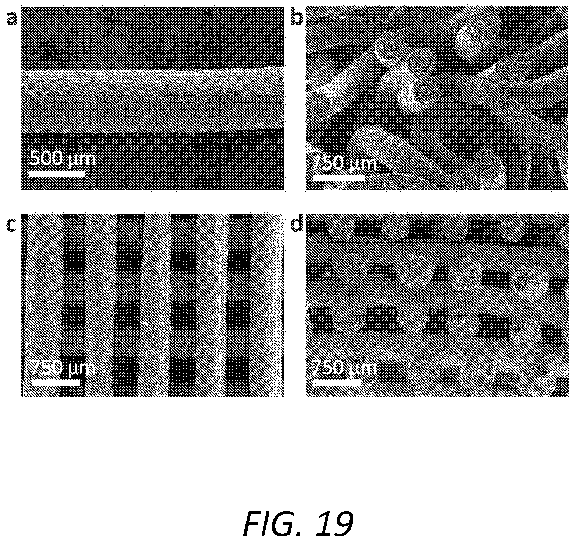

Scanning electron microscopy (SEM) images of solidified dosage forms produced with the apparatus 380 of FIG. 18 include the following: (*a*) single fiber, (*b*) fibrous structure with rannomly or almost randomly arranged fibers, (*c*) top view of an example of a fibrous structure with oriented fibers, and (*d*) front view of an example of a fibrous structure with oriented fibers, as shown in FIG. 19.

Among the advantages of this invention may be one or more of the following. The present invention provides solid pharmaceutical dosage forms with predictable properties and solutions to improve the efficiency in manufacturing solid dosage forms by: (i) reducing the number of unit operations required to produce such forms (this allows to reduce capital cost, the footprint in the facility, labor cost, cost for quality control, etc.), (ii) reducing process time, (iii) reducing solvent requirements, (iv) reducing excipient material requirements (particularly in the case of cellular dosage forms with high drug volume fraction) and (v) providing a process with high process rate (also, particularly in the case of cellular dosage forms with high drug volume fraction, mixing time is reduced). Further, the present invention provides solutions to produce high-quality, aesthetically attractive, melt-processed dosage forms with very rapid drug release (rapid drug release particularly in the case of cellular dosage forms with engineered microstructure). The difficulties in manufacture and development of present granular solid dosage forms can be largely avoided with the dosage forms and manufacturing processes presented here, because the material is processed in liquid form. The drug is dispersed either as molecules or as particles in an excipient that is plasticized by melting or by a solvent. In contrast to processing granular matter, mixing and dispensing liquids is predictive and repeatable, as the streamlines continuously follow a deterministic path, with flow rates that can be calculated from constitutive models. Thus the microstructures produced have increased predictability, repeatability, and if desired, uniformity. Furthermore, the dosage forms presented here allow any of the following (a) to further increase drug release rate and reduce dissolution time by a morphology that allows the dissolution medium to rapidly penetrate the dosage form (b) to reduce the amount of excipient material required (i.e., excipient material cost) by achieving high drug volume fractions of up to about 0.9 with respect to the solid content (c) to reduce mixing time and efforts by mixing in liquid form and achieving high drug volume fractions of up to about 0.9 (d) to increase the process rate at consistently high quality (i.e., low variations in product properties due to the liquid-based processing) (e) to reduce process time and to integrate the process of pharmaceutical manufacturing into a simple, continuous process

## Claims

1. A pharmaceutical solid dosage form comprising:

   a structural assembly of one or more repeatably arranged, extruded structural elements forming a continous microstructure;
   said extruded structural elements comprising at least one pharmaceutically active ingredient and at least one hydrophilic excipient;

said extruded structural elements further comprising segments separated and spaced from adjoining segments by free spacings; and

the free spacings definining one or more nondrug-containing free spaces in said pharmaceutical solid dosage form;

wherein

the one or more extruded structural elements are so arranged that an average 'free spacing' between segments is greater than 1 $\mu$m;

at least one nondrug-containing free space comprises a gas; and

the pharmaceutical solid dosage form comprises an immediate-release dosage form.

2. The dosage form of claim 1, wherein average thickness of the one or more elements is in the range between 5 $\mu$m and 2 mm.

3. The dosage form of claim 1, wherein at least one structural element comprises a fiber or a sheet.

4. The dosage form of claim 1, wherein the one or more structural elements are arranged in such a manner that an interconnected free space exists.

5. The dosage form of claim 1, wherein a minimum number of walls that must be ruptured to obtain an interconnected, continuous cluster of nondrug-containing free space from a surface of the dosage form to any point inside the dosage form is between 0 and 10, for an average element thickness, $h_0$, greater or smaller than 100 $\mu$m.

6. The dosage form of claim 1, wherein at least one excipient comprises a solubility greater than 5g/l in a body fluid.

7. The dosage form of claim 1, wherein at least a drug-containing structural element or a segment thereof is bonded to another drug-containing structural element or another segment of said drug-containing structural element.

8. The dosage form of claim 1, wherein at least one excipient is swellable by a body fluid, and wherein an effective diffusivity of water in said swellable excipient is greater than $1 \times 10^{-11}$ m$^2$/s.

9. The dosage form of claim 8 wherein the swellable excipient comprises a viscosity less than 500 Pa·s upon absorption of the body fluids.

10. The dosage form of claim 1, wherein at least one hydrophilic excipient is selected from the group comprising polyethylene glycol (PEG), polyvinylpyrrolidone (PVP), PEG-PVP copolymer, poloxamer, hydroxypropyl methylcellulose, lauroyl macrogol-32 glycerides, polymethylmethacrylates, polyvinyl alcohol (PVA), PEG-PVA copolymer, polybutylmethacrylat- (2-dimethylaminoethyl)methacrylat-methylmathacrylat-copolymer, polyvinylacetate phthalate, poly(methacrylic acid, ethyl acrylate) 1:1, polyvinyl caprolactam-polyvinylacetate-polyethylene glycol copolymer, or gelatin.

11. The dosage form of claim 1, wherein the nondrug-containing free space comprises a matter selected from the group comprising gas, liquid, or solid, and wherein said matter is partially or entirely removed upon contact with a dissolution fluid.

12. The dosage form of claim 11, wherein the gas comprises at least one of air, nitrogen, $CO_2$, argon, or oxygen.

13. A method of manufacturing the pharmaceutical solid dosage form of claim 1 comprising the steps of:

feeding at least one active ingredient and at least one excipient into an extrusion channel having a cross section extending along its length inside a housing;

mixing said at least one active ingredient and said at least one excipient;

heating the at least one active ingredient and at least one excipient to form a plasticized matrix or combining the at least one active ingredient and at least one excipient with at least one solvent to form a plasticized matrix;

extruding the plasticized matrix towards and through an exit port of the extrusion channel to form at least one structural element; and

structuring at least one structural element to a continuous, structural assembly of one or more repeatably arranged, extruded structural elements;

wherein
said at least one structural element is structured to a continuous, structural assembly of one or more repeatably arranged, extruded structural elements by deposition of said element on a movable stage.

**14.** An apparatus for the manufacture of the pharmaceutical solid dosage forms of claim 1 comprising:

An internally hollow housing having an internal surface encapsulating and defining an extrusion channel having a first end and a second end and a cross section extending axially along its length from said first end to said second end and terminating into an exit port at the second end;
said housing having feeding ports for feeding drug and excipient into the extrusion channel;
said housing further comprising either one or two of items (a) through (b): (a) at least one solvent feeding port for feeding at least one solvent into the extrusion channel and form a plasticized matrix therein, (b) at least one heating element embedded in or attached to the housing to form a plasticized matrix in the extrusion channel;
said apparatus further comprising:

a conveying element for extruding the plasticized matrix towards and through an exit port of the extrusion channel to form at least one structural element; and
a movable stage to structure at least one structural element to a continuous, structural assembly of one or more repeatably arranged, extruded structural elements;

wherein
said at least one structural element is structured to a continuous, structural assembly of one or more repeatably arranged, extruded structural elements by deposition of said structural element on said movable stage.

**Patentansprüche**

**1.** Eine feste pharmazeutische Darreichungsform umfassend:

Ein struktureller Aufbau aus einem oder mehreren wiederholbar angeordneten, extrudierten Strukturelementen, die eine kontinuierliche Mikrostruktur bilden;
die extrudierten Strukturelemente enthalten mindestens einen pharmazeutischen Wirkstoff und mindestens einen hydrophilen pharmazeutischen Hilfsstoff;
die extrudierten Strukturelemente umfassen ferner Segmente, die durch freie Abstände von benachbarten Segmenten getrennt und beabstandet sind; und
die freien Abstände definieren einen oder mehrere nicht wirkstoffhaltige Freiräume in der pharmazeutischen festen Darreichungsform;
wobei
das eine oder die mehreren extrudierten Strukturelemente so angeordnet sind, dass der durchschnittliche "freie Abstand" zwischen den Segmenten größer als 1 $\mu$m ist;
mindestens ein nicht wirkstoffhaltiger Freiraum ein Gas enthält; und
die pharmazeutische feste Darreichungsform eine Darreichungsform mit sofortiger Wirkstofffreisetzung ist.

**2.** Die Darreichungsform nach Anspruch 1, wobei die durchschnittliche Dicke des einen oder der mehreren Elemente im Bereich zwischen 5 $\mu$m und 2 mm liegt.

**3.** Die Darreichungsform nach Anspruch 1, wobei mindestens ein Strukturelement eine Faser oder eine Platte umfasst.

**4.** Die Darreichungsform nach Anspruch 1, wobei das eine oder die mehreren Strukturelemente so angeordnet sind, dass ein zusammenhängender Freiraum besteht.

**5.** Die Darreichungsform nach Anspruch 1, wobei die Mindestanzahl an Wänden, die aufgebrochen werden müssen, um einen zusammenhängenden, kontinuierlichen Cluster von nicht-medikamentenhaltigem Freiraum von einer Oberfläche der Dosierungsform bis zu einem beliebigen Punkt innerhalb der Dosierungsform zu erhalten, zwischen 0 und 10 liegt, bei einer durchschnittlichen Elementdicke, $h_0$, von mehr als oder weniger als 100 $\mu$m.

6. Die Darreichungsform nach Anspruch 1, wobei mindestens ein Hilfsstoff eine Löslichkeit von mehr als 5 g/l in einer Körperflüssigkeit aufweist.

7. Die Darreichungsform nach Anspruch 1, wobei mindestens ein wirkstoffhaltiges Strukturelement oder ein Abschnitt davon an ein anderes wirkstoffhaltiges Strukturelement oder einen anderen Abschnitt des wirkstoffhaltigen Strukturelements gebunden ist.

8. Die Darreichungsform nach Anspruch 1, wobei mindestens ein Trägerstoff durch eine Körperflüssigkeit quellbar ist und wobei die effektive Diffusionsfähigkeit von Wasser in dem quellbaren Trägerstoff größer als $1 \times 10^{-11}$ m$^2$/s ist.

9. Die Darreichungsform nach Anspruch 8, wobei der quellbare Trägerstoff bei Absorption der Körperflüssigkeiten eine Viskosität von weniger als 500 Pa·s aufweist.

10. Die Darreichungsform nach Anspruch 1, wobei mindestens ein hydrophiler Hilfsstoff aus der Gruppe ausgewählt ist, die Polyethylenglykol (PEG), Polyvinylpyrrolidon (PVP), PEG-PVP-Copolymer, Poloxamer, Hydroxypropylmethylcellulose, Lauroyl-Macrogol-32-Glyceride, Polymethylmethacrylate, Polyvinylalkohol (PVA), PEG-PVA-Copolymer, Polybutylmethacrylat-(2-Dimethylaminoethyl)methacrylat-Methylmethacrylat-Copolymer, Polyvinylacetatphthalat, Poly(Methacrylsäure, Ethylacrylat) 1:1, Polyvinylcaprolactam-Polyvinylacetat-Polyethylenglykol-Copolymer oder Gelatine umfasst.

11. Die Darreichungsform nach Anspruch 1, wobei der nicht-medikamentenhaltige freie Raum eine Substanz umfasst, die aus der Gruppe ausgewählt ist, die Gas, Flüssigkeit oder Feststoff umfasst, und wobei die Substanz bei Kontakt mit einer sich auflösenden Körperflüssigkeit teilweise oder vollständig entfernt wird.

12. Die Darreichungsform nach Anspruch 11, wobei das Gas mindestens eines der folgenden Gase umfasst: Luft, Stickstoff, $CO_2$, Argon oder Sauerstoff.

13. Ein Verfahren zur Herstellung der pharmazeutischen festen Darreichungsform nach Anspruch 1 umfassend die folgenden Schritte:

Zuführen mindestens eines Wirkstoffs und mindestens eines Hilfsstoffs in einen Extrusionskanal mit einem sich über seine gesamte Länge erstreckenden Querschnitt innerhalb eines Gehäuses;
Mischen des mindestens einen Wirkstoffs und des mindestens einen Hilfsstoffs;
Erhitzen des mindestens einen Wirkstoffs und des mindestens einen Hilfsstoffs zur Bildung einer plastifizierten Matrix oder Kombinieren des mindestens einen Wirkstoffs und des mindestens einen Hilfsstoffs mit mindestens einem Lösungsmittel zur Bildung einer plastifizierten Matrix;
Extrudieren der plastifizierten Matrix in Richtung und durch eine Austrittsöffnung des Extrusionskanals zur Bildung mindestens eines Strukturelements; und
Strukturieren mindestens eines Strukturelements zu einer kontinuierlichen Struktur aus einem oder mehreren wiederholbar angeordneten, extrudierten Strukturelementen;
wobei
das mindestens eine Strukturelement durch Ablegen des Elements auf einem beweglichen Tisch zu einer kontinuierlichen Struktur aus einem oder mehreren wiederholbar angeordneten, extrudierten Strukturelementen strukturiert wird.

14. Eine Vorrichtung zur Herstellung der pharmazeutischen festen Darreichungsformen nach Anspruch 1, umfassend:

Ein innen hohles Gehäuse mit einer Innenfläche, die einen Extrusionskanal mit einem ersten und einem zweiten Ende umschließt und definiert, der Querschnitt des Extrusionskanals erstreckt sich axial vom ersten zum zweiten Ende und mündet am zweiten Ende in eine Austrittsöffnung;
das Gehäuse weist Zuführöffnungen zum Zuführen von Arzneimittel und Hilfsstoff in den Extrusionskanal auf;
das Gehäuse umfasst ferner einen oder zwei der Punkte (a) bis (b): (a) mindestens eine Lösungsmittelzuführöffnung zum Zuführen mindestens eines Lösungsmittels in den Extrusionskanal und zum Bilden einer plastifizierten Matrix darin; (b) mindestens ein im Gehäuse eingebettetes oder daran befestigtes Heizelement zum Bilden einer plastifizierten Matrix im Extrusionskanal;
die Vorrichtung umfasst ferner:

ein Förderelement zum Extrudieren der plastifizierten Matrix in Richtung und durch eine Austrittsöffnung des Extrusionskanals zur Bildung mindestens eines Strukturelements; und eine bewegliche Plattform zum Strukturieren mindestens eines Strukturelements zu einer kontinuierlichen Struktur aus einem oder mehreren wiederholbar angeordneten,
extrudierten Strukturelementen;
wobei
das mindestens eine Strukturelement durch Ablegen auf dem beweglichen Tisch zu einer kontinuierlichen Struktur aus einem oder mehreren wiederholbar angeordneten, extrudierten Strukturelementen strukturiert wird.

**Revendications**

1. Une forme pharmaceutique solide comprenant:

   un assemblage structurel continu d'un ou plusieurs éléments structuraux extrudés disposés de manière répétitive, formant une microstructure continue;
   lesdits éléments structuraux extrudés comprenant au moins un principe actif pharmaceutique et au moins un excipient hydrophile;
   lesdits éléments structuraux extrudés comprenant en outre des segments séparés et espacés des segments adjacents par des espacements libres; et
   les espacements libres définissant un ou plusieurs espaces libres non médicamenteux dans ladite forme pharmaceutique solide;
   dans laquelle
   le ou les éléments structuraux extrudés sont disposés de manière à ce que l'espacement libre moyen entre les segments soit supérieur à 1 $\mu$m;
   au moins un espace libre non médicamenteux comprend un gaz; et
   la forme pharmaceutique solide comprend une forme pharmaceutique à libération immédiate.

2. La forme pharmaceutique solide selon la revendication 1, dans laquelle l'épaisseur moyenne du ou des éléments est comprise entre 5 $\mu$m et 2 mm.

3. La forme pharmaceutique solide selon la revendication 1, dans laquelle au moins un élément structurel comprend une fibre ou une plaque.

4. La forme pharmaceutique solide selon la revendication 1, dans laquelle le ou les éléments structurels sont disposés de telle manière qu'un espace libre interconnecté existe.

5. La forme pharmaceutique solide selon la revendication 1, dans laquelle un nombre minimum de parois devant être rompues pour obtenir un groupe interconnecté et continu d'espace libre ne contenant pas de médicament à partir d'une surface de la forme posologique jusqu'à n'importe quel point à l'intérieur de la forme posologique est compris entre 0 et 10, pour une épaisseur d'élément moyenne, $h_0$, supérieure ou inférieure à 100 $\mu$m.

6. La forme pharmaceutique solide selon la revendication 1, dans laquelle au moins un excipient comprend une solubilité supérieure à 5 g/l dans un fluide corporel.

7. La forme pharmaceutique solide selon la revendication 1, dans laquelle au moins un élément structurel contenant un médicament ou un segment de celui-ci est lié à un autre élément structurel contenant un médicament ou à un autre segment dudit élément structurel contenant un médicament.

8. La forme pharmaceutique solide selon la revendication 1, dans laquelle au moins un excipient est gonflable par un fluide corporel, et dans laquelle une diffusivité efficace de l'eau dans ledit excipient gonflable est supérieure à $1 \times 10^{-11}$ m$^2$/s.

9. La forme pharmaceutique solide selon la revendication 8, dans laquelle l'excipient gonflable comprend une viscosité inférieure à 500 Pa·s lors de l'absorption des fluides corporels.

10. La forme pharmaceutique solide selon la revendication 1, dans laquelle au moins un excipient hydrophile est choisi dans le groupe comprenant le polyéthylène glycol (PEG), la polyvinylpyrrolidone (PVP), le copolymère PEG-PVP, le poloxamère, l'hydroxypropylméthylcellulose, les lauroyl macrogol-32 glycérides, les polyméthylméthacrylates, l'alcool polyvinylique (PVA), le copolymère PEG-PVA, le copolymère polybutylméthacrylat-(2-diméthylaminoéthyl) méthacrylat-méthylméthacrylat, le phtalate de polyvinylacétate, le poly(acide méthacrylique, acrylate d'éthyle) 1:1, le copolymère polyvinylcaprolactame-polyvinylacétate-polyéthylène glycol, ou la gélatine.

11. La forme pharmaceutique solide selon la revendication 1, dans laquelle l'espace libre ne contenant pas de médicament comprend une matière choisie dans le groupe comprenant un gaz, un liquide ou un solide, et dans laquelle ladite matière est partiellement ou entièrement éliminée lors du contact avec un fluide corporel de dissolution.

12. La forme pharmaceutique solide selon la revendication 11, dans laquelle le gaz comprend au moins l'un des éléments suivants : air, azote, $CO_2$, argon ou oxygène.

13. Une procédé de fabrication de la forme pharmaceutique solide selon la revendication 1, comprenant les étapes suivantes:

introduire au moins un principe actif et au moins un excipient dans un canal d'extrusion dont la section s'étend sur toute sa longueur à l'intérieur d'un boîtier;
mélanger ledit principe actif et ledit excipient;
chauffer ledit principe actif et ledit excipient pour former une matrice plastifiée ou combiner ledit principe actif et ledit excipient avec au moins un solvant pour former une matrice plastifiée;
extruder la matrice plastifiée vers et à travers un orifice de sortie du canal d'extrusion pour former au moins un élément structurel; et
structurer au moins un élément structurel en un assemblage structurel continu d'un ou plusieurs éléments structurels extrudés agencés de manière répétitive;
dans lequel
ledit élément structurel est structuré en un assemblage structurel continu d'un ou plusieurs éléments structurels extrudés agencés de manière répétitive par dépôt dudit élément sur une platine mobile.

14. Un appareil pour la fabrication de formes pharmaceutiques solides selon la revendication 1, comprenant:

Un boîtier creux dont la surface interne encapsule et définit un canal d'extrusion doté d'une première et d'une seconde extrémité, et dont la section transversale s'étend axialement sur toute sa longueur, de ladite première à ladite seconde extrémité, et se termine par un orifice de sortie à la seconde extrémité;
Ledit boîtier étant doté d'orifices d'alimentation pour l'introduction du médicament et de l'excipient dans le canal d'extrusion;
Ledit boîtier comprenant en outre un ou deux des éléments (a) à (b): (a) au moins un orifice d'alimentation en solvant pour introduire au moins un solvant dans le canal d'extrusion et y former une matrice plastifiée; (b) au moins un élément chauffant intégré ou fixé au boîtier pour former une matrice plastifiée dans le canal d'extrusion;
L'appareil comprenant en outre:

Un élément de transport pour extruder la matrice plastifiée vers et à travers un orifice de sortie du canal d'extrusion afin de former au moins un élément structurel; et
Une platine mobile pour structurer au moins un élément structurel en un assemblage structurel continu d'un ou plusieurs éléments structurels extrudés disposés de manière répétitive;
dans lequel
ledit au moins un élément structurel est structuré en un assemblage structurel continu d'un ou plusieurs éléments structurels extrudés agencés de manière répétitive par dépôt dudit élément structurel sur ledit plateau mobile.

FIG. 1A
(prior art)

FIG. 1B
(prior art)

FIG. 2A
(prior art)

FIG. 2B
(prior art)

FIG. 3A                    FIG. 3B

FIG. 4

*FIG. 5*

FIG. 6

FIG. 7

**FIG. 8**

a

160

D

Drug
100

91

$h_0$

H

Excipient of drug
containing solid
120

Biocompatible solid
122

b

160

D

Drug
100

92

H

h

Excipient of drug
containing solid
120

Biocompatible solid, liquid, or gas
124

c

D

Drug
100

93

H

h

160

Excipient of drug
containing solid
120

Fast eroding solid
126

*FIG. 9*

a

$D$

$H$

Fast eroding solid

Drug containing solid

$\phi_{fes} = 0.1$

b

$D$

$H$

$\lambda_f$

$h$

Fast eroding solid

Drug containing solid

$\phi_{fes} = 0.5$

*FIG. 10*

190

Preparation of a particulate mixture of solid drug particles and an excipient (one or several) at a determined drug-to-excipient mass (or volume) ratio (192).

The particulate mixture is filled into a screw-type extruder (194).

The particulate mixture is plasticized/fluidized in the extruder under heat or via a solvent. The plasticized mixture is mixed and pressurized (195).

The fluidic mixture is then pressed through a die by the extrusion screw  (196).

After passing through the die the extruded material is cooled or dried (197).

The elements are arranged/assembled to the final dosage form, preferably in a mold or on a surface (198).

## FIG. 11

FIG. 12

**FIG. 13**

Deposition of component 1

Deposition of component 2

*FIG. 14*

EP 3 368 010 B1

*210*

Preparation of a particulate mixture of solid drug particles and an excipient (one or several) at a determined drug-to-excipient mass (or volume) ratio (212)

↓

The particulate mixture is filled into a screw-type extruder (214)

↓

The particulate mixture is plasticized/fluidized in the extruder under heat or via a solvent. The plasticized mixture is mixed and pressurized. (215)

↓

The fluidic mixture is then pushed forward with the extrusion screw, the pressure of the material is increased, and gas bubbles are injected. (216)

↓

The pressurized fluidic mixture is pressurized further and is then pressed through a die by the extrusion screw. (217)

↓

After passing through the die the extruded material is cooled or dried, (218), preferably in a mold (219)

# FIG. 15

**FIG. 16**

EP 3 368 010 B1

**FIG. 18**

FIG. 19

**REFERENCES CITED IN THE DESCRIPTION**

**Patent documents cited in the description**

- US 62246470
- US 90789116
- US 62360546
- US 62377068
- US 14907891 B
- US 7112016